# EUROPEAN PATENT SPECIFICATION

(11) **EP 3 837 551 B1**
(45) Date of publication and mention of the grant of the patent: **21.02.2024**
(21) Application number: 19756341.4
(22) Date of filing: 16.08.2019
(51) Int. Cl.: G01N 33/68

(54) **MODULATORS OF THE FUNCTION OF FACTOR H-RELATED PROTEIN 1 AND USES THEREOF IN THE CONTROL OF INFLAMMATION**
MODULATOREN DER FUNKTION VON FAKTOR H-VERWANDTEM PROTEIN 1 UND DEREN VERWENDUNG BEI DER KONTROLLE VON ENTZÜNDUNGEN
MODULATEURS DE LA FONCTION DE LA PROTÉINE 1 APPARENTÉE AU FACTEUR H ET LEURS UTILISATIONS DANS LE CONTRÔLE DE L'INFLAMMATION

(30) Priority: 16.08.2018 DE 102018120016
(43) Date of publication of application: 23.06.2021
(73) Proprietor: Medizinische Hochschule Hannover, 30625 Hannover (DE)
(72) Inventor: ZIPFEL, Peter, 07743 Jena (DE); SKERKA, Christine, 07743 Jena (DE); HERR, Sarah Maria, 30625 Hannover (DE)
(74) Representative: Krauss, Jan
(86) International application number: PCT/EP2019/072039
(87) International publication number: WO 2020/035603

(56) References cited:
- WO-A1-2014/118552
- WO-A1-2017/109208
- WO-A2-2008/008986
- ÁDÁM I. CSINCSI ET AL: "FHR-1 Binds to C-Reactive Protein and Enhances Rather than Inhibits Complement Activation", THE JOURNAL OF IMMUNOLOGY, vol. 199, no. 1, 1 July 2017 (2017-07-01), pages 292-303, XP055497261, US ISSN: 0022-1767, DOI: 10.4049/jimmunol.1600483
- IRMSCHER SARAH ET AL: "FHR1 triggers the inflammasome and drives sterile inflammation in human diseases", MOLECULAR IMMUNOLOGY, PERGAMON, GB, vol. 102, 11 September 2018 (2018-09-11), page 164, XP085471690, ISSN: 0161-5890, DOI: 10.1016/J.MOLIMM.2018.06.099
- SARAH IRMSCHER ET AL: "Serum FHR1 binding to necrotic-type cells activates monocytic inflammasome and marks necrotic sites in vasculopathies", NATURE COMMUNICATIONS, vol. 10, no. 1, 4 July 2019 (2019-07-04), XP055621507, DOI: 10.1038/s41467-019-10766-0

## Description

The present invention relates to modulators of the function of factor H related protein 1 (FHR1).

### Background of the invention

Cellular stress is a spontaneous event triggered by injury or infection and is a characteristic of inflammatory diseases such as anti-neutrophil cytoplasmic antibody-associated vasculitis (AAV) (1) and atherosclerosis (AS) (2, 3). Necrotic cells act as unmodified danger-associated molecular patterns (DAMPs), which activate innate immunity and, together with membrane-anchored molecules, recruit and activate the inflammasome in immune cells such as monocytes or neutrophils (4). Secretion of pro-inflammatory cytokines IL-1β, IL-6, IL-8, and TNFα recruits phagocytic leukocytes and activates stem cells to replace dead cells (5). However, sustained immune responses can seriously damage host tissues and thus cause various diseases.

Sterile inflammation occurs in acute conditions such as ischemia reperfusion injury and crystal-induced arthritis, and also with chronic diseases such as particle-induced lung diseases and atherosclerosis. Although the details are not fully understood, generally, non-microbial activators trigger such inflammation. AAV is a systemic autoimmune disease characterized by autoantibodies specific for proteinase 3 (PR3) and myeloperoxidase (MPO) expressed by neutrophils. Pathophysiology often shows pauci-immune crescentic glomerulonephritis (1). Activation of the alternative complement pathway amplifies the recruitment, priming, and activation of neutrophils, thereby creating a self-amplifying inflammatory loop that results in destructive and necrotizing vascular injury. In AAV, small- and medium-sized blood vessels, such as those in the kidney and lung, are infiltrated by immune cells and eventually destroyed (6).

AS is a chronic vascular disease and its expression as coronary artery disease (CAP) the most common cause of death in developed countries. Lipid deposits form in regions of arteries with disturbed blood flow, leading to formation of atherosclerotic plaques comprising necrotic cells, which trigger sterile inflammation. Rupture of plaques can cause stroke or myocardial infarction. Patients with AS show high activation of the inflammasome 3 (NLRP3) in the aorta (2, 3).

Complement senses DAMPs and pathogen-associated molecular patterns (PAMPs). Complement activation destroys microbes and induces phagocytosis and clearance mechanisms also of damaged, dead human cells. Factor H (FH), the main regulator of the alternative complement pathway, is crucial in the protection of self surfaces from the damaging effects of complement (7).

US 2009-0215895 relates to compounds which are useful therapeutic and prophylactic molecules useful in the treatment and prophylaxis of a range of conditions including cancers, protein kinase C(PKC)- or NFkB-related- or - associated conditions, cardiovascular conditions, pain, inflammatory conditions, vascular or immunological conditions such as diabetes, neurological conditions and infection by a range of viruses or prokaryotic or eukaryotic organisms. One of the numerous molecules as mentioned is FHR1.

WO 2008/008986 discloses methods for determining a human subject's propensity to develop, therapeutic or prophylactic compounds for treating a vascular disorder and/or age-related macular degeneration (AMD) or inhibiting its development, and methods of treating patients to alleviate symptoms of the disease, prevent or delay its onset, or inhibit its progression. The methods are based on the discovery that persons with a genome having a deletion of the CFHR-1 and/or CFHR-3 gene, which normally lie on human chromosome 1 between DNA encoding CFH and CFHR-4, are at reduced risk of developing AMD, and elevated risk of developing vascular disease such as aneurysm.

WO 2012/112955 discloses screening methods to identify compounds that bind to and reduce the expression or biological activity of a either CFHR1 or CFHR3.

WO 2014/118552 discloses agents and compounds which can be used to modulate the activity of the complement system, novel biological targets associated with such modulation, and pharmaceutical compositions, medicaments and methods of treatment for use in preventing, ameliorating or treating diseases that are characterized by inappropriate complement activity. These diseases include age-related macular degeneration (AMD), meningitis, renal disease, autoimmune disease and inflammation. Therapeutic antibodies and screening assays for identifying agents useful in treating these diseases are disclosed.

Irmscher S et al. (Irmscher S, Brix SR, Zipfel SLH, Halder LD, Mutlutürk S, Wulf S, Girdauskas E, Reichenspurner H, Stahl RAK, Jungnickel B, Wiech T, Zipfel PF, Skerka C. Serum FHR1 binding to necrotic-type cells activates monocytic inflammasome and marks necrotic sites in vasculopathies. Nat Commun. 2019 Jul 4;10(1):2961) describe human serum factor H-related protein FHR1 as a dominant trigger of inflammation. In vitro, this protein selectively binds to necrotic cells via its N-terminus; in addition, it binds near necrotic glomerular sites of AAV patients and necrotic areas in atherosclerotic plaques. FHR1, but not factor H, FHR2 or FHR3 strongly induces inflammasome NLRP3 in blood-derived human monocytes, which subsequently secrete IL-1β, TNFα, IL-18 and IL-6. FHR1 triggers the phospholipase C-pathway via the G-protein coupled receptor EMR2 independent of complement. Moreover, FHR1 concentrations of AAV patients negatively correlate with glomerular filtration rates and associate with the levels of inflammation and progressive disease. The data highlight an unexpected role for FHR1 during sterile inflammation, may explain why FHR1-deficiency protects against certain diseases, and identifies potential targets for treatment of auto-inflammatory diseases.

The central function of the factor H related protein FHR1 remains unclear to date. New strategies for treating and controlling diseases involving FHR1 and its signal cascade are desirable.

The invention is defined in the appended claims.

According to a first aspect thereof, the above object of the invention is solved by a method for identifying a compound that modulates sterile inflammation through the modulation of the interaction of FHR1 with EMR2/ADGRE2 in a cell, comprising the steps of a) contacting at least one of FHR1, an EMR2/ADGRE2 binding fragment of FHR1, a cell expressing FHR1, and/or a cell expressing an EMR2/ADGRE2 binding fragment of FHR1 with at least one compound that potentially modulates the interaction of FHR1 with EMR2/ADGRE2 in a cell b) identifying a modulation of the binding of FHR1 or said fragment to EMR2/ADGRE2 in the presence of said at least one compound, and c) identifying a compound as identified in step b) as specifically modulating sterile inflammation..

Preferred is the method according to the present invention, wherein said modulation is selected from a decrease or an increase of said binding of FHR1 to EMR2/ADGRE2.

It was found in the context of the present invention the human serum protein called "factor H related protein 1" (FHR1) binds to necrotic cells in order to strongly induce and activate monocytes. This interaction of FHR1 with the necrotic cells is mediated by the N-terminus of the FHR1 molecule. Therefore, this region was identified as a target in order to develop inhibitors of this function (e.g. Fab fragments as disclosed below). The binding of FHR1 seems to take place after a dimerization of said molecule.

These monocytes produce and secrete pro-inflammatory cytokines in order to attract additional immune cells, thus causing an inflammation response. Once the damage is reverted, usually the inflammation stops, and homeostasis occurs. Nevertheless, in a number of autoinflammatory conditions, a permanent stimulation can be observed, leading to a so-called training of the immune cells. This training causes a trained immunity of the immune cells which leads to much stronger inflammation. This inflammation can cause organ damage and failure, in particular in case of diseases with permanent cellular damages, like arteriosclerosis and vasculitis, but also cirrhosis. The protein as identified provides a link between cellular damages and the development and maintenance of sterile inflammation.

Therefore, FHR1 itself and its signaling cascade as identified including the receptor offer the possibility to induce this process even further (cancer cells) or to inhibit it (arteriosclerosis, vasculitis, cirrhosis, trauma, etc.). Three inhibitor classes were already tested in *in* vitro experiments, a) a natural competitor of FHR1, in particular in the context of dimerization, FHR2, b) a monoclonal antibody against FHR1, and c) an FHR1 receptor blocker.

In contrast to the well-studied vital functions of FH, the central function of the factor H related protein FHR1 remains unclear to date. FHR1 comprises five short consensus repeats (SCRs) but lacks the regulatory domains found in FH. *In vitro* assays revealed that FHR1 inhibits the terminal complement pathway in the absence of FH (8). In addition, FHR1 competes with FH to regulate FH inhibitory activity (9, 10). However, the composition and concentration of FHR1 suggest a more specific role in immunity. The N-terminal short consensus repeats (SCRs), named SCR1-2, within FHR1 are 36% and 45% homologous to FH/SCR6-7, respectively, and contain a hybridization domain (10, 11). The C-terminal SCR3-5 domains are 100/97%, 100%, and 98% identical to FH/SCR18-20 respectively, and bind C3b, C3d, and heparin (8). FHR1 circulates in serum as homodimers and forms heterodimers with FHR211, which are contained in so-called FHR1-related protein lipoprotein particles (FALPs) (12). Genetic deletion of a chromosomal fragment comprising *CFHR3-CFHR1* genes (ΔFHR1/3) confers protection against IgAN (13) and AMD (14), but susceptibility to systemic lupus erythematosus (SLE) (15) and HUS (16). The reason for these opposing associations between FHR1 and different diseases is still unclear, although likely ascribed to an as-yet-unknown function of FHR1. Here, the inventors identified a new role for FHR1 in sterile inflammation.

Further preferred is a method according to the present invention, wherein said compound is selected from small molecular drugs.

Further preferred is a method according to the present invention, further comprising the step(s) of testing said compound as identified for its activity to induce or reduce sterile inflammatory responses, for example by detecting cytokine IL-1 β, IL-6, IL-18, C-reactive protein (CRP), and/or TNFα.

Even further preferred is a method according to the present invention that further comprises a computational analysis of and optimization of the compound based on the structure, in particular the three-dimensional and/or crystal structure of FHR1 and/or EMR2/ADGRE2.

According to a second aspect thereof, the object of the present invention is solved by the use of an isolated cell expressing FHR1, and/or expressing an EMR2/ADGRE2 binding fragment thereof, wherein said cell optionally expresses EMR2/ADGRE2 and/or an FHR1 binding fragment thereof, as a screening tool for screening for a compound that modulates the interaction of FHR1 with EMR2/ADGRE2 on/in a cell.

Thus, a method for identifying a compound that modulates sterile inflammation through the modulation of at least one of the expression, the biological activity and/or the interaction of FHR1 with EMR2/ADGRE2 in a cell was designed. The method comprises steps of:
Contacting at least one of FHR1, an EMR2/ADGRE2 binding fragment of FHR1, a cell expressing FHR1, and/or a cell expressing an EMR2/ADGRE2 binding fragment of FHR1 with at least one compound that potentially modulates the interaction of FHR1 with EMR2/ADGRE2 in a cell. This part of the assay generally screens for compounds that interact with FHR1 or an EMR2/ADGRE2 binding fragment of FHR1.

In the next step, the method then involves an identifying of a modulation of the binding of FHR1 or said fragment to EMR2/ADGRE2 or the binding of EMR2/ADGRE2 or said fragment to FHR1 in the presence of said at least one compound. The modulation is selected from a) an increase of said binding, and/or b) a stabilization of said binding, and c) a decrease of binding of FHR1 and/or EMR2/ADGRE2 in said cell as disclosed herein.

In a next step, a compound as identified in step b) is further identified as specifically modulating sterile inflammation as disclosed herein.

Preferred is a screening assay that screens for compounds that are specific for FHR1, and induce and/or increase/enhance the activity thereof in the specific binding to EMR2/ADGRE2.

It was surprisingly found by the present inventors that the binding pair FHR1-EMR2/ADGRE2 or fragments or the components thereof, i.e. FHR1 and/or EMR2/ADGRE2 provide valuable tools for therapeutic approaches in order to treat or prevent a disease or condition characterized by a release of the pro-inflammatory cytokines IL-1β, IL-6, IL-18, CRP, and/or TNFα, sterile inflammation, cancer, arteriosclerosis, vasculitis, such as antibody-associated vasculitis (AAV), cirrhosis, trauma, reperfusion injury, transplantation, diabetes, Morbus Alzheimer, multiple sclerosis, IgA nephropathy, C3-glomerulopathy, and age related-macular degeneration. The inventors found it conceivable to target said components in human disease without affecting vital functions.

In the context of the present invention, the term "FHR1" shall be understood as also indicating/representing the mammalian (in particular mouse) ortholog/homolog of the human FHR1 gene and/or protein and/or mRNA. Also, the term shall comprise the complete FHR1 polypeptide or fragment(s) as described herein, such as the C-terminus of FHR1/SCR3-5 or N-terminus FHR1/SCR1-2 of the FHR1 polypeptide. The term also covers FHR1 in its dimeric form, in its form dimerized with FHR2 polypeptide, and in different preparations, such as in the cellular context, purified from the cell. The amino acid sequence of CHFR (aliases: HFL1, HFL2, CFHL, FHR1, H36, Complement Factor H-Related Protein 1, Complement Factor H-Related 1, H36-1, and H36-2) can be, e.g. found at UNIPROT 003591.

Similarly, "EMR2/ADGRE2" shall be understood as also indicating/representing the mammalian (if present, identified) ortholog/homolog of the human EMR2/ADGRE2 gene and/or protein and/or mRNA. Also, the term shall comprise the complete EMR2/ADGRE2 polypeptide or functional fragment(s) as described herein, such as FHR1-binding fragment. The term also covers EMR2/ADGRE2 in different preparations, such as in the cellular context, purified from the cell. The amino acid sequence of EMR2 (aliases: ADGRE2, and CD132) can be, e.g. found at UNIPROT Q9UHX3.

The term "contacting" in the present invention means any interaction between the potentially binding substance(s) with FHR1 and/or EMR2/ADGRE2, whereby any of the components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip, and FHR1 and/or EMR2/ADGRE2 (or a functional part thereof) or the bound complex thereof are subsequently contacted with such a chip.

Preferably, said modulation is selected from a decrease or an increase of said binding of FHR1 to EMR2/ADGRE2.

In the context of the present invention, the term "biological activity" shall preferably include the cellular functions of FHR1, EMR2/ADGRE2, the complex of FHR1 with EMR2/ADGRE2, and/or the fragments thereof in the cell in the process of sterile inflammation, for example their function(s) in the signaling cascade and/or in inducing other factors. One specific preferred example is the dimerization of FHR1, as apparently mediated by the N-terminal region thereof.

Further preferably, said identifying comprises a method selected from rtPCR, immunoprecipitation and measuring the induction or reduction of inflammation in said cell, for example by detecting cytokine IL-1β. Other cytokines to be detected can be IL-6, IL-18, CRP, and/or TNFα. Respective assays are also known to the person of skill, and disclosed below.

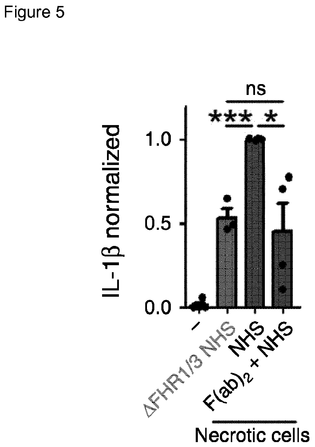

More preferred is a method according to the present invention, wherein said compound is selected from the group consisting of a peptide library, a combinatory library, a cell extract, a "small molecular drug", an antisense oligonucleotide, an siRNA, and an antibody, in particular an antibody against FHR1 or fragment thereof specifically interfering with the binding of FHR1 to EMR2/ADGRE2, FHR2 or binding fragment thereof, and a receptor blocker, such as an NLRP3 inhibitor. Particularly preferred are antibody fragments against FHR1 that reduce inflammation (see Figure 5). The inventors conclude that these F(ab)2-Fragments prevent binding of FHR1 to necrotic cells and therefore protects from an FHR1 induced inflammatory response. Consequently monoclonal antibodies against FHR1 could be a promising therapeutic approach.

In the context of the present invention, a "small molecular drug" or "small molecule" is one that has a molecular weight of below 2000 Da, preferably of below 1000 Da, optimally in the range of between 600 and 200 Da.

Preferred is a method according to the present invention, wherein said FHR1 and/or said EMR2/ADGRE2 is immobilized, such as, for example, on a microtiter plate, beads or a chip, like a glass slide.

Further preferred is a method according to the present invention, wherein said method comprises the use of human peripheral blood monocytes, normal human serum (NHS), and/or lipopolysaccharide (LPS). These conditions are preferred/envisioned for all methods according to the present invention as disclosed herein (e.g. also the evolutionary methods, as disclosed below).

According to the invention, said cell is selected from the group of recombinant host cells expressing FHR1 or the EMR2/ADGRE2 binding fragment thereof, wherein said recombinant host cells optionally express EMR2/ADGRE2, yeast cells, and recombinant bacterial cells.

Preferred is a method according to the present invention, wherein said EMR2/ADGRE2 binding fragment of FHR1 comprises the C-terminus of FHR1/SCR3-5 or the N-terminus FHR1/1-2 of the FHR1 polypeptide. The FHR1 and/or EMR2/ADGRE2 or the binding fragment(s) thereof employed in a method of the present invention can be a full length protein or a fragment with N/C-terminal and/or internal deletions.

Another aspect is directed at a method according to the present invention, further comprising testing said compound(s) as identified for its/their activity to induce or reduce sterile inflammatory responses. Respective assays are known to the person of skill, and can be taken from the respective literature.

Preferred is a method according to the present invention, wherein said FHR1 and/or EMR2/ADGRE2 and/or the fragments thereof are suitably labeled. Measuring of binding of the compound to FHR1 and/or EMR2/ADGRE2 and/or the fragments thereof can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of FHR1 and/or EMR2/ADGRE2 and/or the fragments thereof or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to FHR1 and/or EMR2/ADGRE2 and/or the fragments thereof. The effect of the binding of the compound or the activity of FHR1 and/or EMR2/ADGRE2 and/or the fragments thereof can also be measured indirectly, for example, by assaying an enzymatic activity of FHR1 and/or EMR2/ADGRE2 and/or the fragments thereof after binding.

As a further step the method according to the present invention said method can furthermore comprise a computational optimization based on said structure of the binding pair FHR1-EMR2/ADGRE2. Respective methods are known to the person of skill.

As a further step after measuring the binding of a potentially interacting compound and after having measured at least two different potentially interacting compounds at least one compound can be selected, for example, on grounds of the measured binding activity or on grounds of the detected increase of FHR1 and/or EMR2/ADGRE2 binding activity.

The thus selected binding compound can then in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with a method of the present invention, i.e. they are contacted with FHR1, dimeric FHR1,and/or EMR2/ADGRE2 and/or the fragments thereof and subsequently binding of the modified compounds to the FHR1 and/or EMR2/ADGRE2 and/or the fragments thereof is measured. In this step, both the binding per se can be measured and/or the effect of the function of the FHR1 and/or EMR2/ADGRE2 and/or the fragments thereof like, e.g. the binding to FHR1 and/or EMR2/ADGRE2and/or the fragments thereof can be measured. If needed the steps of selecting the binding compound, modifying the binding compound, contacting the binding compound with FHR1 and/or EMR2/ADGRE2 and/or the fragments thereof and measuring the binding of the modified compounds to the protein(s) can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity.

According to another aspect thereof, the object of the present invention is solved by the use of an isolated cell expressing FHR1, and/or expressing an EMR2/ADGRE2 binding fragment thereof, wherein said cell optionally expresses EMR2/ADGRE2 and/or an FHR1 binding fragment thereof, as a screening tool for screening for a compound that modulates the interaction of FHR1 with EMR2/ADGRE2 on/in a cell.

The cell can be a prokaryotic or eukaryotic cell, and any genetic expression constructs can be present extrachromosomally or integrated into the chromosome. The polypeptides can be expressed in the form of a fusion protein, for example together with an enzymatically active moiety as reporter-construct, in order to be able to detect the expression product. Preferred host cells are derived from cells selected from the skeletal muscle, liver, adipose tissue, heart, pancreas, kidney, breast tissue, ovarian tissue, and/or hypothalamus. Thus, preferred is a screening tool according to the present invention, wherein said cell is selected from necrotic cells, cancer cells; recombinant host cells expressing said FHR1 and/or EMR2/ADGRE2 and/or binding fragments thereof; yeast cells; and (recombinant) bacterial cells.

Preferred is the use according to the present invention, wherein said FHR1 and/or EMR2/ADGRE2 and/or the fragments thereof are immobilized and/or labeled, as also described above.

Further preferred is the use according to the present invention, wherein said EMR2/ADGRE2 binding fragment of FHR1 comprises the C-terminus of FHR1/SCR3-5 or N-terminus FHR1/SCR1-2 of the FHR1 polypeptide.

Disclosed is a method for manufacturing a pharmaceutical composition for treating or preventing a disease or condition characterized by a release of the pro-inflammatory cytokine IL-1β, cancer, arteriosclerosis, vasculitis, such as antibody-associated vasculitis (AAV), cirrhosis or trauma, comprising the steps of performing the method according to the present invention, and formulating said compound as identified into a pharmaceutical composition.

The interacting compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routs of application like, for example, intravenous solution, sprays, band-aids or pills.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

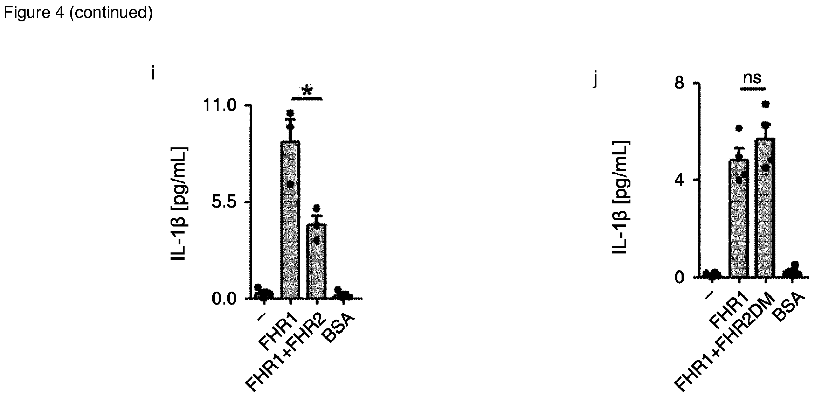

Inflammation is a fundamental cellular response to harmful stimuli such as pathogens, trauma, and necrosis. In these cases, transient inflammation leads to clearance and healing, thereby maintaining cellular homeostasis. However, sustained immune responses can cause autoinflammation, in which innate immune responses play the primary pathophysiological role (28). Here, the inventors identified a new human inducer of inflammation, FHR1, which binds preferentially to oxidized LDL on necrotic human cells and strongly induces the NLRP3 inflammasome in monocytes via GPCR EMR2 exposed to NHS (Figure 4j). FHR1 deficiency, which is present in 4-7% of Caucasians (27), leads to a substantial reduction in IL-1β secretion (about 50%) in response to necrosis. Similarly, AAV patients harboring ΔFHR1/3 have low serum concentrations of IL-1β as well as CRP. These data demonstrate a new and dominant function of FHR1 in necrosis-triggered inflammation, which may explain why ΔFHR1/3 protects against autoinflammatory diseases such as IgAN (13) and AMD (14). Both diseases are characterized by cell stress and pathological inflammatory process with involvement of the NLRP3 inflammasome (29).

Immobilized FHR1 (as well as the murine homolog FHRB), but not FHR2, FHR3, FH, or the spliced variant of FH, FHL-1, induces the NLRP3 inflammasome in monocytes, which subsequently secrete pro-inflammatory cytokines. IL-1β and TNFα are central cytokines in immune cell communication and activation and as such recruit innate immune cells to the site of infection and cell damage. IL-6 triggers CRP release and IL-18 is responsible for generating interferon-γ (IFN-y) and for increasing the cytolytic activity of natural killer cells and T cells. To induce IL-1 β, FHR1 binds to oxidized lipids on necrotic cells via its N-terminal domain, linking the proinflammatory features of oxidized lipids (30, 31) to NLRP3 activation. Upon binding FHR1 likely changes its conformation such that the C-terminus becomes exposed and mediates the inflammatory response. As both the C-terminal domains of FHR1 (SCR3-5) and FH (SCR19-20) induce IL-1β secretion by monocytes, this suggests that the C-terminal domains of both proteins are hidden or protected when they are circulating in human plasma. Indeed, FHR1 is present primarily in so-called FALPs (12), which may shield the inflammatory activity of FHR1. Similarly, FH with a circular structure was previously described, in which the C-terminus is folded back (32). Like FH, FHR1 can bind to the TED domain in C3b via the C-terminal SCR domains (33). When bound to C3b, FHR1 lacks pro-inflammatory activity, confirming the C-terminus as the main inflammatory actor in FHR1. In summary, FHR1 can activate the NLRP3 inflammasome in immune cells by binding directly via its N-terminus to oxidized lipids on necrotic cells. FHR1 also competes with FH when bound to C3b. FHR1 activates the NLRP3 inflammasome in monocytes via the PLC and Ca2+ activation pathways. RNAseq data derived from FHR1-induced monocytes revealed upregulation of G protein-coupled receptor EMR2/ADGRE2, which induces inflammatory responses in macrophages (21, 34) and suggested involvement of this receptor in FHR1 function. Specific inhibition of EMR2 blocked FHR1 induced IL-1β release of monocytes. EMR2 activity in FHR1 function is supported by the fact that EMR2 is highly expressed in foamy macrophages in atherosclerosis (35).

Necrosis plays an important role in AAV and treatment of inflammation through aggressive immunosuppression is the established therapy (36, 37). Also, in AS recent trials follow inhibition of inflammation (38). ΔFHR1/3 was detected in 3.5% of AAV cases. IL-1β concentrations were not increased in ΔFHR1/3 AAV patients; indeed, the concentrations were low and comparable to those measured in healthy controls. Moreover, also the acute phase protein and inflammation marker CRP, which increases in response to IL-6, was significantly lower (p<0.001) in ΔFHR1/3 AAV samples compared to patients harboring FHR1. These results confirm the pro-inflammatory function of FHR1 and demonstrate a fundamental role in sterile inflammation. ΔFHR1/3 was detected in 2.8% of acute AS cases (n=141). CRP concentrations were much lower in ΔFHR1/3 AS patients than AS patients with FHR1. Both diseases are characterized by necrosis and inflammation, including NLRP3 activation and elevated IL-1β serum concentrations (24), but the initial triggers in AAV and AS are different.

Genetically, ΔFHR1/3 protects against IgA nephropathy (13) and AMD (39, 40) and is a risk for atypical hemolytic uremic syndrome (aHUS) (41) and SLE (27), as well as rheumatoid arthritis (42) (all of which are associated with inflammation). Thus, it is tempting to speculate that in AMD and IgAN FHR1 is the major driver of inflammation and that ΔFHR1/3 protects against these diseases; however, the pathological mechanisms underlying these diseases are more complex. That said, patients with these chronic diseases need to be evaluated to support the pro-inflammatory role of FHR1 and that symptoms arise from established lesions.

Most importantly, the inventors' data support direct intervention with FHR1 function to inhibit inflammation in human diseases like AAV and AS, to reduce progression and even induce regression. Therefore, targeting FHR1 or members of the FHR1 pathway to control inflammation in these (and other) autoinflammatory diseases presents as a promising therapeutic approach for future intervention.

Persistent inflammation is a hallmark of many human diseases, including anti-neutrophil cytoplasmic antibody-associated vasculitis (AAV) and atherosclerosis (AS). Here, the inventors describe a novel and dominant trigger of inflammation: human serum factor H-related protein FHR1. *In vitro,* this protein selectively binds to necrotic cells via its N-terminus; in addition, it binds to necrotic glomerular cells of AAV patients and necrotic areas in AS plaques. FHR1, but not factor H, FHR2, FHR3, or FHL-1, strongly induces inflammasome 3 in blood-derived human monocytes, which subsequently secrete IL-1β, TNFα, IL-18, and IL-6. FHR1 triggers the phospholipase C-pathway via the G-protein coupled receptor EMR2 independent of complement. Moreover, FHR1-deficient AAV (11/314) patients present with low serum concentrations of IL-1β and both deficient AAV and AS (3/124) patients low C-reactive protein. These new data highlight an unexpected role for FHR1 during sterile inflammation and may explain why FHR1-deficiency protects against certain diseases. Also, the study identifies potential new targets for treatment of autoinflammatory diseases.

The present invention will now be further described in the examples with reference to the accompanying figures, nevertheless, without wanting to be limited thereto. In the Figures:

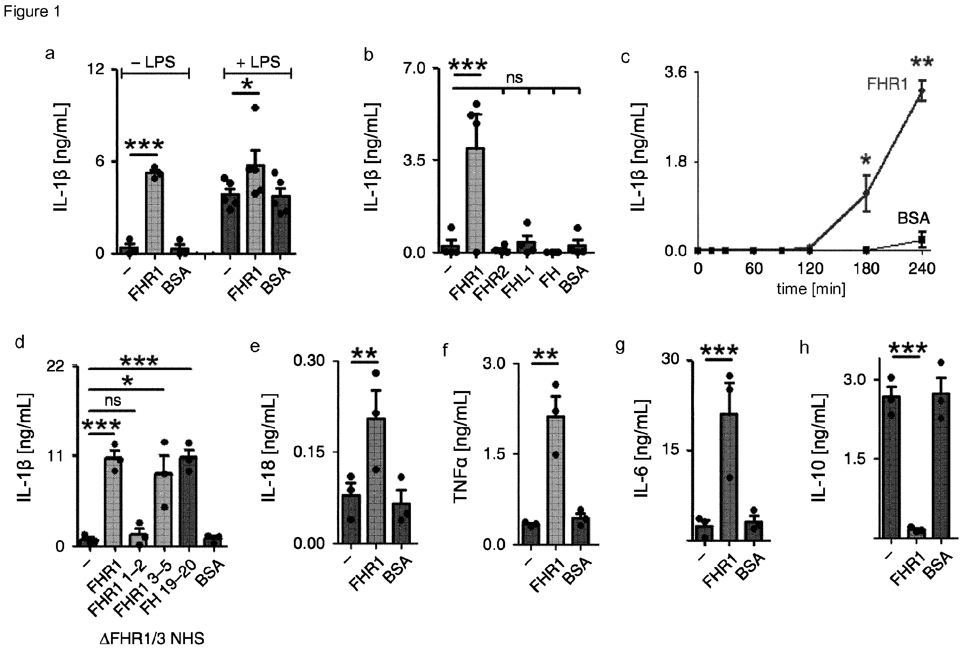

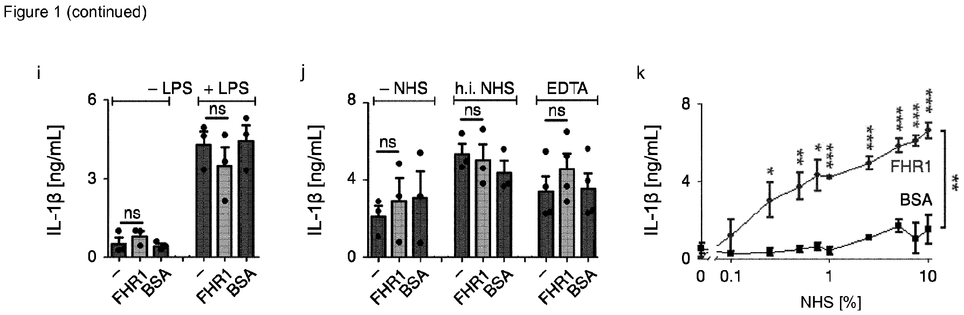

Figure 1 shows that immobilized FHR1 induces inflammation. (a) Immobilized FHR1 induces IL-1β secretion by monocytes and increases IL-1β production by LPS-stimulated monocytes in the presence of NHS. (b) In contrast to immobilized FHR1, FHR2, FHL-1, FH, and BSA fail to induce IL-1β secretion. (c) Immobilized FHR1 triggers IL-1β release from monocytes as early as 3 h after the start of incubation. (d) FHR1 SCR3-5 and FH SCR19-20, but not FHR1 SCR1-2, trigger IL-1β release by monocytes exposed to ΔFHR1/3 NHS. (e) Immobilized FHR1 induces secretion of IL-18, (f) TNFα, and (g) IL-6. (h) Immobilized FHR1 inhibits secretion of IL-10 by LPS-stimulated monocytes. (i) Unbound FHR1 fails to increase secretion of IL-1β by monocytes. (j) In the absence of NHS or in the presence of heat-inactivated (h.i.) and EDTA-inactivated NHS, immobilized FHR1 does not induce IL-1β secretion. (k) FHR1 induces secretion of IL-1β upon exposure to 0.25% NHS. Increasing the concentration of NHS increases IL-1β release. Data in a-k represent the mean ± SEM of three to five independent experiments with different donor cells. *p≤0.05, **p≤0.01, ***p≤0.001 (unpaired two-tailed t-test).

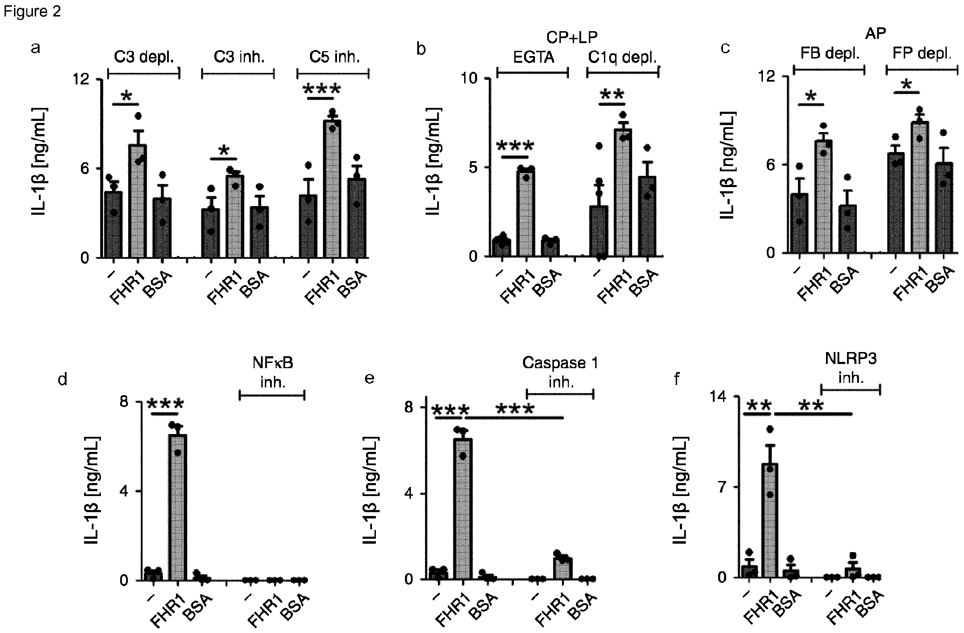

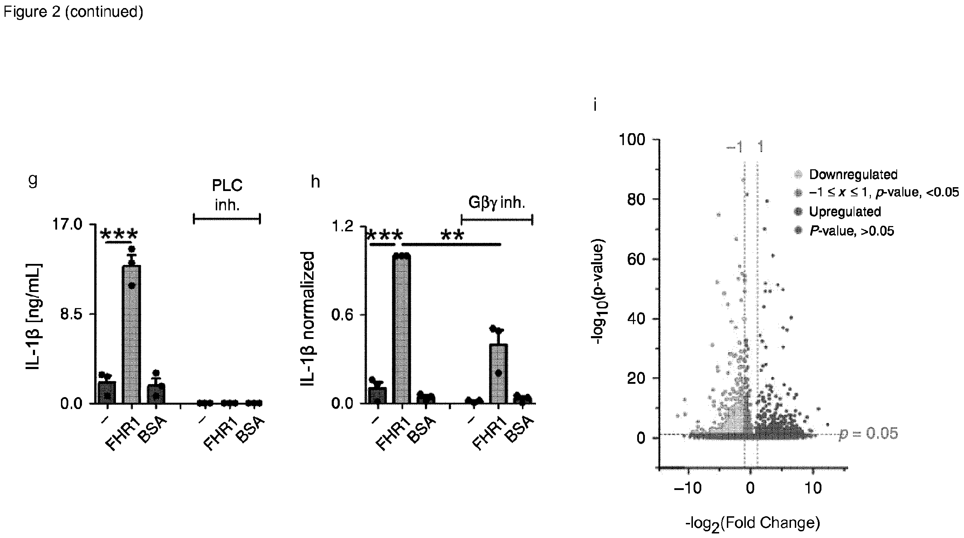

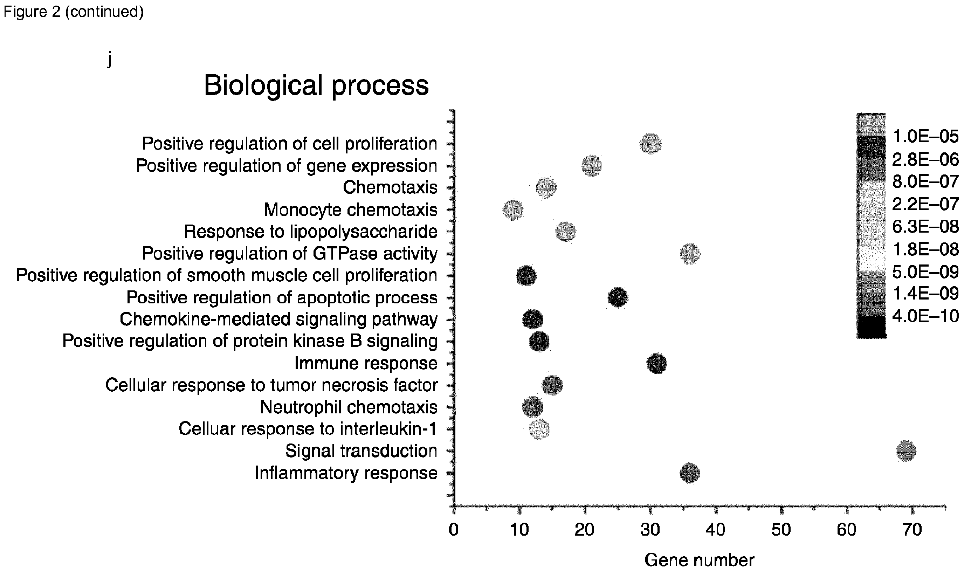

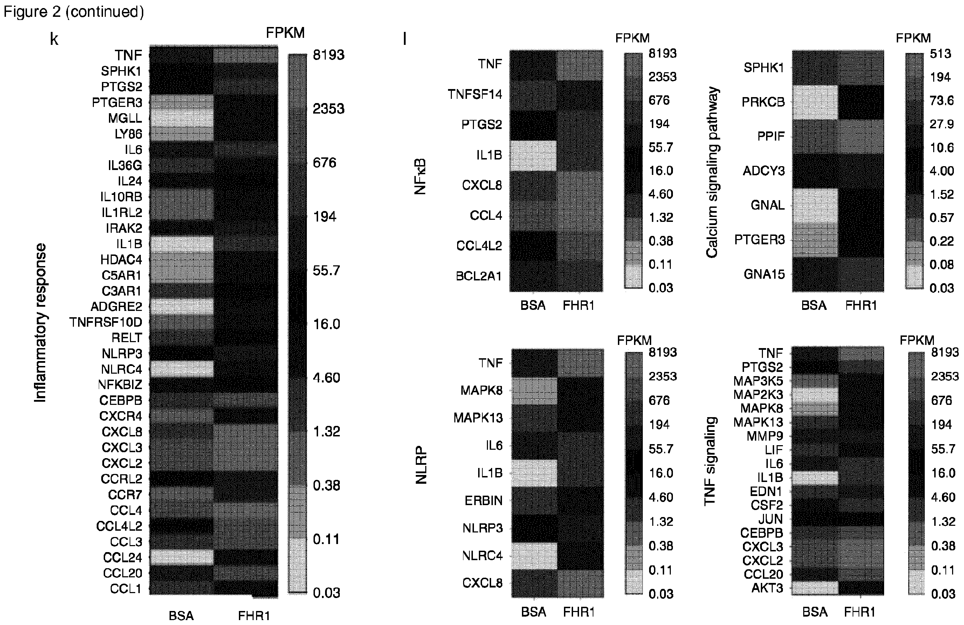

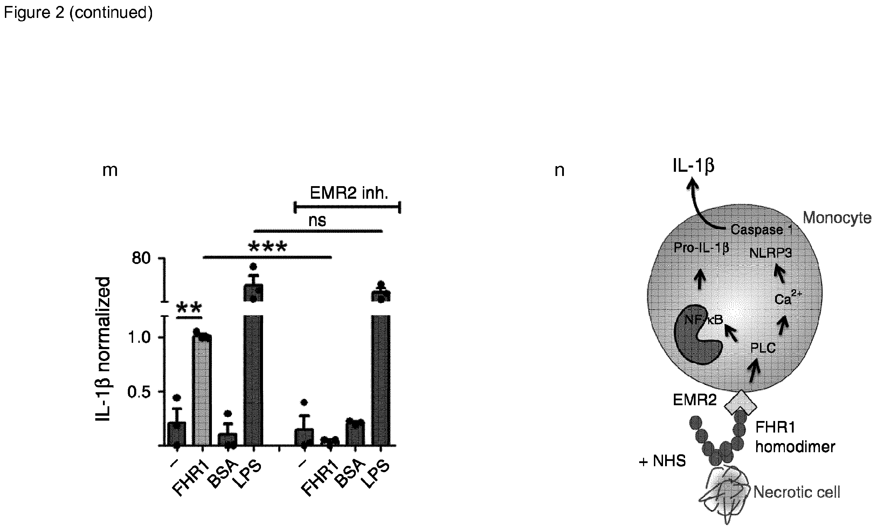

Figure 2 shows that FHR1 activates the NLRP3 inflammasome in monocytes via the PLC pathway. (a) Immobilized FHR1 increases secretion of IL-1β in LPS-treated monocytes exposed to C3-depleted, C3-inhibited (Compstatin), or C5-inhibited (Eculizumab) NHS. (b) FHR1 stimulates secretion of IL-1β by EGTA-treated or C1q-depleted NHS, which blocks the classical (CP) and lectin pathways (LP) activation, respectively, as well as in (c) factor B (FB)- and factor P (FP)-depleted NHS (in which the alternative pathway (AP) is inhibited). (d) IL-1β induction by FHR1 is blocked when NF-κB is inhibited by BAY 11-7085. (e) IL-1β secretion is blocked by a caspase-1 inhibitor (VX-765), (f) by a NLRP3 inhibitor (MCC950), or (g) by a PLC inhibitor (U73122). (h) Blocking Gβγ subunit of GPCRs with Gallein reduces IL-1β induction by FHR1 by about 65% after 4 h incubation. (i) Genes differentially expressed in monocytes exposed for 4 h to FHR1 in NHS. (j) Biological processes involved in FHR1 induction (GO classification). (k) Inflammatory genes and (I) signaling pathways induced in monocytes by FHR1. (m) IL-1β induction by FHR1 after 4 h is blocked by inhibition of EMR2 (αEMR2). (n) Schematic overview of FHR1-induced IL-1β secretion by monocytes. Data in a-m represent the mean ± SEM of three to five independent experiments with cells from different donors. *p≥0.05, **p≤0.01, ***p≤0.001 (unpaired two-tailed t-test).

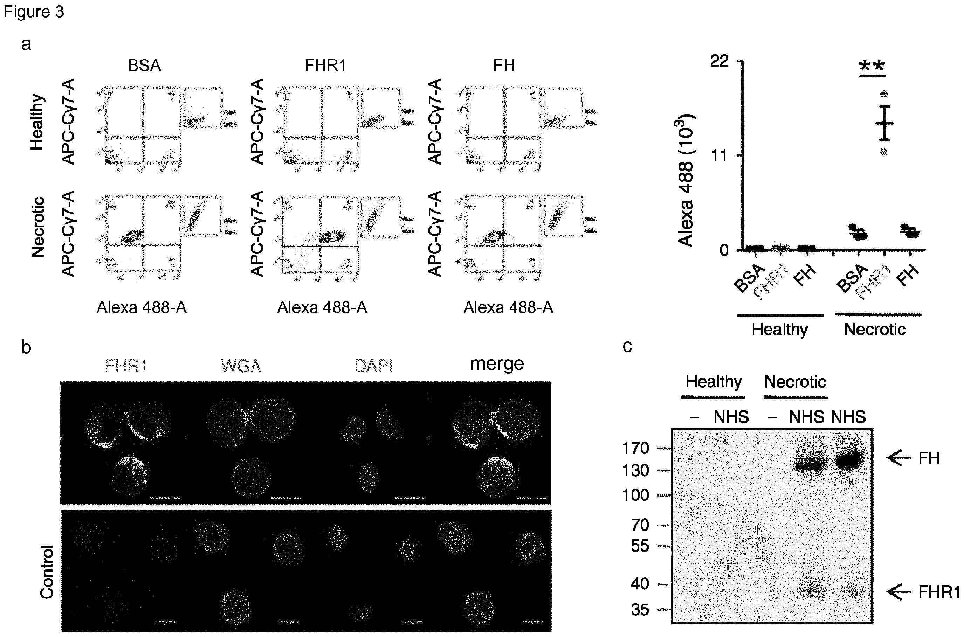

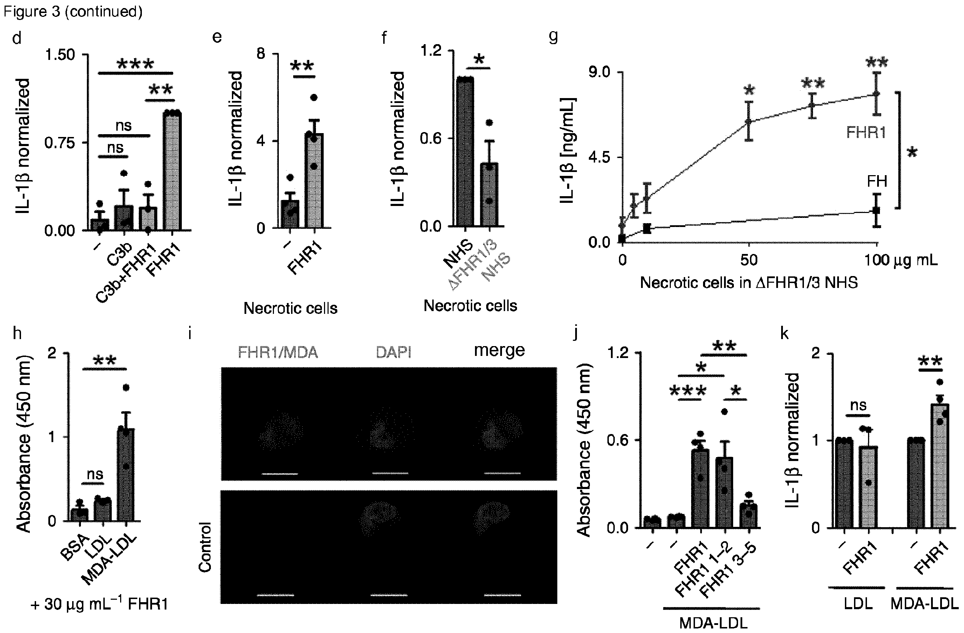

Figure 3 shows that FHR1 triggers IL-1β, IL-6, TNFα release by monocytes. (a) FHR1, but not FH, binds to necrotic HUVECs, as shown by flow cytometry. (b) FHR1 (green) binds to specific spots in the absence of membrane staining by WGA (Texas Red) on necrotic cells. DNA (blue) is stained with DAPI. Scale=10 µm. (c) FHR1 and FH from NHS bind to necrotic, but not healthy, cells. One representative western blot of three is shown. (d) FHR1 bound to C3b does not induce IL-1β release by monocytes. (e) FHR1 bound to necrotic HUVECs triggers IL-1β release by monocytes. (f) IL-1β release in response to necrotic HUVECs is by about 60% reduced upon exposure to ΔFHR1/3 NHS than upon exposure to NHS. (g) Reconstitution of FHR1 in ΔFHR1/3 NHS results in increased binding of FHR1 to necrotic cells and increased IL-1β release by monocytes. FH has no effect. (h) FHR1 binds to MDA-LDL, but not to LDL or BSA. (i) FHR1 binds to MDA epitopes on necrotic cells, as shown by a proximity ligation assay (PLA). Red dots denote FHR1-MDA complexes. DNA is stained with DAPI (blue). (j) FHR1 binds with its N-terminus FHR1 SCR1-2 to MDA-LDL. (k) FHR1 bound to MDA-LDL, but not LDL, increases IL-1β release by monocytes. Data in a and d-k represent the mean ± SEM of three to four independent experiments with different cell donors. *p≥0.05, **p≤0.01, ***p≤0.001 (unpaired two-tailed t-test).

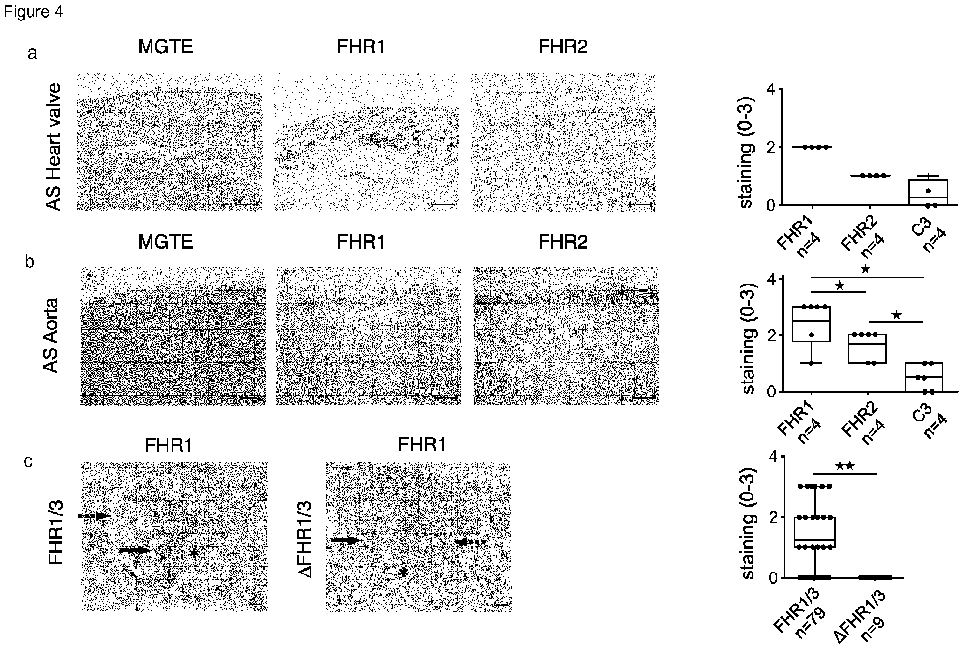

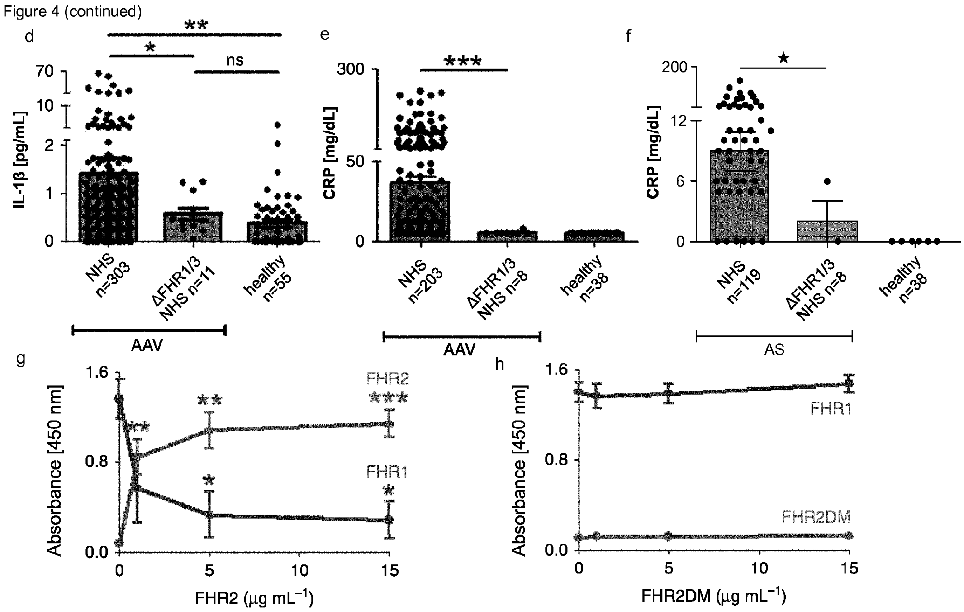

Figure 4 shows the in *vivo* functions of FHR1. (a) FHR1 (red) binds to atherosclerotic plaques in human heart valve and (b) human arteries in AS patients. FHR2 is present in the intima of arteries. Low amounts of C3 are detected. *p≤0.0>5, **p≤0.01, ***p≤0.001 (c) In AAV, FHR1 staining appears on fibrinous necrosis in the glomerulus (asterisk), surrounded by necrotic cells. Healthy tissue (arrow) and unaffected cells (discontinued arrow) do not bind FHR1. Glomeruli in ΔFHR1/3 AAV patients are FHR1 negative. Statistical analysis is performed using Wilcoxon Signed Rank Test. *p≥0.05, **p≤0.01, ***p≤0.001 (d) IL-1β and (e) CRP concentrations in sera of AAV patients or (f) AS patients harboring FHR1/3 are significantly higher than in ΔFHR1/3 AAV patients or healthy individuals (unpaired two tailed t-test with Welch's correction). *p≥0.05, **p≤0.01, ***p≤0.001 (g) FHR1 from NHS binds to immobilized FHR1 SCR1-2 and is replaced by FHR2 in a dose-dependent manner, but (h) not by the dimerization mutant FHR2DM. (i) FHR1 added to necrotic cells increases IL-1β release by monocytes exposed to ΔFHR1/3 NHS, which is reduced by about 50% when the same concentration of FHR2 is added but not when (j) the dimerization mutant FHR2DM is added. Data in g-j represent the mean ± SEM of three independent experiments with three different cell donors. *p≥0.05, **p≤0.01, ***p≤0.001 (unpaired two tailed t-test).
Figure 5 shows that variable chain F(ab)2 of monoclonal FHR1 antibody reduces inflammation. Incubation of necrotic cells with monocytes in presence of NHS leads to a strong release of IL-1 β, which is reduced by about 50% in ΔFHR1/3 NHS. Monoclonal antibody (generated F(ab)2-Fragments of this antibody, which contain the variable, but not the constant chains of the antibody) against the N-terminus of FHR1 (binding region to necrotic cells) reduced the IL-1β release of monocytes in response to necrotic cells in NHS by 50%. Inhibition reached the same level IL- β as ΔFHR1/3 NHS.

### EXAMPLES

### Material and Methods

### Cell growth conditions

Human umbilical vein endothelial cells (HUVEC, ATCC CRL-1730) were grown in DMEM (Lonza) with FBS (10%, PAA), ultraglutamine (1%, Lonza) and gentamicin sulfate (Lonza) at 37°C and CO2 (5%). Murine alveolar MH-S macrophages (ATCC CRL-2019) were grown in RPMI 1640 (Lonza) supplemented with heat inactivated fetal bovine serum (10% FBS, ATCC), sodium-pyruvate (1 mM, Lonza), ultraglutamine (1%) and gentamicin sulfate (50 mg/mL) at 37°C and CO₂ (5%). Cells were passaged every second day until passage 30. Cells were detached with trypsin (0.25%)/EDTA (0.02%) (Biochrome). Necrosis was induced by incubating the cells for 30 - 35 min at 63°C. Normal human serum (NHS) was prepared from FHR1/3 sufficient as well as FHR1/3 deficient (ΔFHR1/3 NHS) blood from healthy volunteers, as determined by PCR (Lo, H.J. et al. Nonfilamentous C. albicans mutants are avirulent. Cell 90, 939-949 (1997)) and Western blot analysis. After coagulation blood was centrifuged (10 min, 2.000 x g, 4°C), and NHS kept frozen in aliquots at -80°C. *C. albicans* cph1Δ/efg1Δ (Lo, H.J. et al. Nonfilamentous C. albicans mutants are avirulent. Cell 90, 939-949 (1997); Wartenberg, A. et al. Microevolution of Candida albicans in macrophages restores filamentation in a nonfilamentous mutant. PLoS genetics 10, e1004824 (2014)), which cannot form hyphae, was grown overnight in YPD medium (D-glucose (2%), peptone (1%), yeast extract in H₂O (5%)) at room temperature.

### Isolation of human monocytes

Bicoll (14 mL, Biochrom) was overlayed by a mixture of DPBS (5 mL, Lonza) and buffy coat (30 mL), derived from healthy male donors. After centrifugation (20 min, 550 x g) the PBMC layer was washed with DPBS (5 min, 160 x g). Bicoll and wash steps were repeated twice. Afterwards the pellet was resuspended in IMDM (25 mL, Thermo Scientific) and used for overlaying Percoll (46%) (GE Healthcare Life Sciences) in IMDM. After centrifugation (20 min, 550 x g) the PBMC layer was washed with DPBS. Monocytes were selected according to the 27 protocol of the human Monocyte Isolation Kit II (MiltenyiBiotec). Monocyte experiments were performed in complete medium composed of 10% FBS and gentamicin sulfate in IMDM (Thermo Fisher Scientific).

### Isolation of murine bone marrow-derived macrophages

All animal experiments were approved by the appropriate institutional and governmental committees for animal welfare. Bone marrow cells were isolated from 10-19 weeks old male mice (C57BL/6) and differentiated to macrophages according to the protocol of Francke et al. (Francke, A., Herold, J., Weinert, S., Strasser, R.H. & Braun-Dullaeus, R.C. Generation of mature murine monocytes from heterogeneous bone marrow and description of their properties. The journal of histochemistry and cytochemistry: official journal of the Histochemistry Society 59, 813-825 (2011)) with modifications. Briefly, femurs and tibiae of the mice were rinsed by piercing with needles (size 14) using RPMI 1640 supplemented with heat-inactivated FCS (10%), ultraglutamine (2 mM), and gentamicin sulphate (25 µg/mL, Lonza). After gentle pipetting and straining through 70 µM nylon cells from each mouse were seeded into 75 cm² ultra low attachment flask (Corning) with growth medium (20 mL) supplemented with recombinant mouse M-CSF (25 ng/mL, Biolegend) for 5 days at 37°C with 5% CO₂. Adherent cells were harvested and washed in DPBS. Blood was collected from 10-25 weeks old mice (C57BL/6) by cardiac puncture in hirudin S-Monovette (Sarstedt). Serum was collected by centrifugation (2000 × g, 10 min, 4°C) and stored at -20°C.

### Proteins

Recombinant FHR1, FHR1 SCR1-2, FHR1 SCR3-5, and FHR2 were expressed in *Pichia pastoris* and purified as previously described (Heinen, S. et al. Factor H-related protein 1 (CFHR-1) inhibits complement C5 convertase activity and terminal complex formation. Blood 114, 2439-2447 (2009). Siegel, C. et al. Complement factor H-related proteins CFHR2 and CFHR5 represent novel ligands for the infection-associated CRASP proteins of Borrelia burgdorferi. PloS one 5, e13519 (2010)). Recombinant FHL1, FHR3, and FH SCR19-20 were expressed in baculovirus expression system as previously described (Kühn, S. & Zipfel, P.F. The baculovirus expression vector pBSV-8His directs secretion of histidine tagged proteins. Gene 162, 225-229 (1995). Hellwage, J. et al. Functional properties of complement factor H-related proteins FHR-3 and FHR-4: binding to the C3d region of C3b and differential regulation by heparin. FEBS letters 462, 345-352 (1999). Hellwage, J., Skerka, C. & Zipfel, P.F. Biochemical and functional characterization of the factor-H related protein 4 (FHR-4). Immunopharmacology 38, 149-157 (1997)). BSA was purchased by AppliChem and factor H (FH) from Complement Technology. Full length FHRB (pCMV3-insert-Myc) was purchased from Sino Biological Inc. and the coding sequences restricted by Kpn1/53 Xba1, cloned into piCZαB (Invitrogen) expression vector and expressed in *P. pastoris.* Polyclonal FHRB antiserum was generated by immunizing rabbits with recombinant FHRB (Davids Biotechnologie GmbH). A dimerization mutant of FHR2 was generated by *in vitro* mutagenesis of the CFHR2 containing piCZα expression vector (Eberhardt, H.U. et al. Human factor H-related protein 2 (CFHR2) regulates complement activation. PloS one 8, e78617 (2013)) using Quick Change Multi Site-Directed Mutagenesis Kit (Agilent Technologies) and the primer CAAGTTCCTACAGGGGAAGTTTTCT**C**TTACT**A**CTGTGA**G**A**G**AATTTTGTGT CTCCTTCAAAATCCT (SEQ ID NO: 1) (mutations in bold and underlined). Mutagenesis was confirmed by sequencing. The His-tagged protein was expressed in *P. pastoris* strain X33 and purified by nickel chelate affinity chromatography. All proteins were evaluated by silver gel and western blot analyses.

### Cytokine experiments

FHR1, FHR2, FHR3, FHL1, FH, and BSA (each 50 µL of 5 µg/mL) were coated on a High Binding ELISA plate (Sarstedt) for 1 h at 37°C. Afterwards proteins were incubated with 1 × 10⁵ monocytes/well in complete medium with or without 10% NHS and with or without stimulation with 5 ng/mL LPS. After 20 h incubation at 37°C and CO₂ (5%) cytokines in the supernatant were measured according to the protocol of the human cytokine ELISA kits: IL-1β, IL-6 and IL-10 (ThermoFisherScientific), IL-18 (Peprotech), IL-18 (R&D Systems) and TNFα (BioLegend). The absorbance was measured by microplate reader (TECAN, Safire). For dose dependent studies 50 µL of 0.6, 1.25, 2.5, and 5 µg/mL of FHR1 were used for coating. For time dependent studies FHR1 or BSA were incubated with monocytes for 0, 15, 30, 60, 90, 120, 180, and 240 min.

Fragments FHR1 SCR1-2, FHR1 SCR3-5 or FH SCR19-20 (each 50 µL of 5 µg/mL) were coated and incubated with monocytes in ΔFHR1/3 NHS (10%) or with LPS treated monocytes in NHS (10%). FHRB (5 µg/mL) or DPBS were coated to a microtiter plate and incubated with mouse monocytes (1 × 10⁵) in normal mouse serum (10%) and 5% CO2 with or without MCC950 (10 µM, InvivoGen) for 20 h at 37°C. IL-1β was measured in the supernatant using a mouse IL-1β kit (BioLegend). Testing FHR1 activity in serum, plates were blocked with BSA prior incubation of monocytes with FHR1 (5 µg/mL) or BSA in serum with or without LPS.

In another assay NHS was heat inactivated 30 min at 56°C or inactivated by addition of EDTA (10 mM, Roth). To measure dose dependency, monocytes were incubated in 0, 0.1, 0.25, 0.5, 0.75, 1, 2.5, 5, 7.5, 10 % NHS with IMDM without FBS. For inhibition studies: C1q, C3, factor B (FB) and factor D (FD) depleted NHS was purchased by Complement Technology. C3 inhibition was performed with Compstatin (15 µM, Tocris Bioscience), C5 with Eculizumab (50 nM, Alexion Pharmaceuticals), CR3 by Simvastatin (20 µM, Sigma-Aldrich) and C3aR by trifluoroacetate salt (TAS) (200 nM, Sigma-Aldrich). Classical and lectin pathways were inhibited with EGTA (10 mM, Sigma-Aldrich). NF-κB was blocked with BAY 11-7085 (30 µM, Sigma-Aldrich), TLR2 with pAb hTLR-2 (10µg/mL, InvivoGen), TLR4 with LPS-RS Ultrapure (22.2 µg/mL, InvivoGen), TLR6 with pAb hTLR-6 (10 µg/mL, InvivoGen), Dectin 1 with WGP Soluble (1 µg/mL, InvivoGen), FcR with FcR Blocking Reagent (2 µL/mL, MiltenyiBiotec), CD14 with FITC hCD14 antibody (10µg/mL, BioLegend), CD36 with mAb hCD36 (5 µg/mL, Hycultec) and the human RAGE receptor with RAGE mouse anti-human clone 176902 (10 µg/mL, R&D Systems).

The NLRP3-inflammasome was blocked by addition of MCC950 (10 µM, Invivogen), Caspase-1 by VX-765 (50 µg/mL, InvivoGen), K+ efflux by Glybenclamide (25 µg/mL, InvivoGen), Cathepsin B by Cathepsin B inhibitor (1µM, Santa Cruz Biotechnology) and PLC by U73122 (10 µM, Abcam) or 1,10-Phenanthroline monohydrate (200 µM, AppliChem). Gβγ subunit of GPCR was blocked with Gallein (10 µM, Tocris) and EMR2 receptor by αEMR2 (10 µg/mL, R&D Systems) and IL-1β was measured after 4h incubation. The IL-1β response to FHR1 bound to C3b 104 was determined after C3b (5 µg/mL) was coated for 1h at 37°C. C3b was blocked with blocking buffer I (200 µL) for 1h at 37°C and incubated with FHR1 (10 µg/mL) for 2h at 37°C. Each step was followed by washing (4 x) with PBST. Afterwards monocytes (1 × 10⁵) were added and incubated for 20 h at 37°C.

Healthy and necrotic (treated 35 min at 63°C) HUVECs (each 1 × 105) were incubated with FHR1 or BSA (each 10 µg) for 20 min at 37°C. After washing with DPBS (1%BSA), HUVEC (1.5 × 104) cells were incubated with monocytes (6 × 104) in CM with NHS (10%) for 20 h at 37°C and CO2 (5%). The supernatant was collected for IL-1β measurement. In addition, monocytes (1 × 105) were incubated with necrotic HUVECs (5 × 104) in NHS or ΔFHR1/3 NHS (each 10%) in CM. Afterwards ΔFHR1/3 NHS was reconstituted with FHR1 (0, 5, 10, 50, 75 or 100 µg/mL) or FH (10 or 100 µg/mL).

To investigate the influence of FHR2, monocytes (1 × 105) were incubated with necrotic HUVECs (5 x 104) in ΔFHR1/3 NHS (5%) in CM with addition of BSA (3 µg) or FHR1 (3 µg) with or without FHR2 or FHR2DM (each 3 µg). FHR1 binding to low density lipoprotein (LDL) and Malondialdehyde-modified LDL (MDA-LDL) (Cell Biolabs) was performed by coating LDL (Cell Biolabs) or MDA-LDL (each 5 µg/mL) for 1h at 37°C, blocked with blocking buffer I (200 µL, AppliChem) for 1h at 37°C and incubated with FHR1 (10 µg/mL) for 2h at 37°C. Incubation with monocytes was followed by cytokine measurement as described above.

### RNA purification, PCR, and RNA Sequencing

FHR1 (50 µL of 5 µg/mL) or BSA was immobilized and incubated with monocytes (1 × 10⁵) in CM with or without NHS (10%) for 4 h at 37°C. RNA was extracted using total RNA purification kit (NORGEN) and converted to cDNA by High Capacity RNA6 to-cDNA Kit (Thermo Fisher Scientific). Quantitative PCR (StepOnePlus^{™} Real-Time PCR System, Thermo Fisher Scientific) was performed with cDNA derived from total RNA (0.25 ng according to NanoDrop ND-1000 Spectrophotometer (peqlab Biotechnologie), PerfeCTA SYBR Green Fast mix Low Rox (Quanta Bio) and primers
ACTB forward 5'GCTAAGTCCTGCCCTCATTT'3, (SEQ ID NO: 2);
ACTB reverse 5'GTACAGGTCTTTGCGGATGT'3, (SEQ ID NO: 3);
IL-1β forward CTCTCACCTCTCCTACTCACTT`3, (SEQ ID NO: 4);
IL-1β reverse TCAGAATGTGGGAGCGAATG'3, (SEQ ID NO: 5);
   and
TNFα forward 5'CCAGGGACCTCTCTCTAATCA'3, (SEQ ID NO: 6);
   and
TNFα reverse 5'TCAGCTTGAGGGTTTGCTAC`3, (SEQ ID NO: 7).

Actin beta was used as endogenous control. Data were analyzed with Expression Suite Software version 1.1 and StepOne^{™} Software v2.3. RNA from FHR1 (0.85 µg) treated monocytes from 4 different donors were mixed (total 3.4 µg) and sequenced by LCSciences. Data were analysed using DAVID 6.7 and Panther 9.0 software.

### LDH measurement

FHR1 (50 µL of 5 µg/mL) or BSA or DPBS were coated to a microtiter plate for 1 h at 37°C. Afterwards the proteins were incubated with monocytes (105) in IMDM with NHS (0.25%) for 20h at 37°C in CO2 (5%). After 19 h lysis buffer (10 µL) was added to three wells (triplicates) and LDH release measured after incubated for 45 min at 37°C. Supernatants (50 µL) were incubated with the reaction mixture of Pierce LDH Cytotoxicity Assay Kit (50 µL, Thermo Scientific) for 30 min at RT. The reaction was stopped by addition of stop solution (50 µL) and the absorbance was measured at 490 nm.

### ROS measurement

FHR1 (50 µL of 5 µg/mL), BSA or DPBS were coated to a microtiter plate for 1 h at 37°C. Proteins were incubated with monocytes (105) in CM with or without 10% NHS. As positive control monocytes (10⁵) were incubated with C. *albicans* cph1Δ/efg1Δ (5 × 104). After 20 h incubation at 37°C and CO₂ (5%) CellROX^{™} Deep Red Reagent (2 µL/mL, Thermo Scientific) was added and the absorbance measured at Ex/Em 480/515-550 nm.

### Protein binding to HUVEC

Binding of FHR1 to healthy and necrotic HUVEC (each 3 × 105) was determined by incubation with FHR1, FH or BSA (each 10 µg) for 20 min at 37°C in DPBS (1% BSA). Cells were stained with mAbC02 (1:500) (Oppermann, M. et al. The C-terminus of complement regulator Factor H mediates target recognition: evidence for a compact conformation of the native protein. Clinical and experimental immunology 144, 342-352 (2006) and Alexa Fluor 488 donkey anti mouse IgG (1:500, Invitrogen) with Viability Dye eFluor^{®} 780 (1:10000, eBioscience), each for 20 min at 4°C in DPBS (1% BSA). Between each step the cells were washed two times with DPBS (1% BSA) and fluorescence was measured via flow cytometry.

FHR1 fragments SCR1-2 and SCR 3-5 (each 50 µl of 60 µg/mL) were incubated to necrotic cells (5 × 10⁵) for 30 min at 37°C in DPBS (1% BSA). The cells were stained with Penta His antibody (1:500, QUIAGEN) and Alexa 488 donkey α-mouse (1:500), Viability Dye eFluor^{®} 780 (1:10.000) each for 20 min at 4°C in DPBS (1% BSA). Between each step cells were washed two times with DPBS (1% BSA) and fluorescence measured by flow cytometry.

Binding of FHR1 and FH from NHS was evaluated by incubating healthy or necrotic HUVECs (each 1 × 10⁶) in 50% NHS (diluted in DPBS) for 30 min at 37°C. After extensive washing, cells were lysed 30 min on ice in lysis buffer (70 µL) pH 7.4 with Triton X-100 (1% v/v) (Sigma) and Protease Inhibitor 185 Cocktail (Roche) in DPBS. Roti^{®}-Load 2 (Roth) 13.5 µL was added to each sample (40 µL) and heated for 10 min at 95°C. Samples (40 µl) were separated via SDS-PAGE and immunoblotted using polyclonal FHR1 antiserum (1:1000) and HRP-conjugated α-rabbit antibody (1:1000, Dako). FHR1 (10 µg/mL) was again bound to necrotic HUVECs (45 min at 65°C) for 30 min at 37°C and cells were stained with polyclonal FHR1 antiserum (Skerka, C. & Zipfel, P.F. Complement factor H related proteins in immune diseases. Vaccine 26, I9-I14 (2008)) (1:1.000) for 30 min at RT. Cells were incubated with Alexa FluorTM488 donkey anti rabbit IgG (1:500, Invitrogen), DAPI (10 µg/mL, Sigma), and WGA Texas Red (1:100, Invitrogen) for 30 min at RT. Between each step, cells were washed twice with 1% BSA in DPBS. Images were captured using LSM 710 (Zeiss) with ZEN 2009 software.

### Proximity ligation assay

FHR1 (20 µg/mL) was incubated with necrotic HUVECs (45 min at 65°) at 37°C for 60 min with shaking at 700 rpm. Washed cells (5 × 105) were seeded onto Poly-L lysine (Sigma-Aldrich) coated diagnostic slides, type PTFE (Carl Roth) and incubated with polyclonal rabbit FHR1 antiserum11 (1:500) and monoclonal mouse MDA-LDL antibody E014 (Tsiantoulas, D. et al. Circulating microparticles carry oxidation-specific epitopes and are recognized by natural IgM antibodies. Journal of lipid research 56, 440-448 (2015)) (1:100). Control cells were incubated with polyclonal rabbit FHR1 antiserum11 (1:500) and mouse IgM κ Isotype Ctrl antibody (Biolegend) (1:50). PLAassay was performed according to manufacturer's protocol provided with Duolink In Situ Red Starter Kit Mouse/Rabbit (Sigma-Aldrich). Images were captured using LSM 710 with ZEN 2011.

### FHRB binding to mouse alveolar macrophages

Healthy as well as necrotic (30 min at 63°C) mouse alveolar macrophages (each 0.5 × 10⁵) were incubated with FHRB or BSA (each 10 ug) for 20 min at 37°C followed by with rabbit FHRB antiserum, generated to the recombinant protein 211 (1:500) and Alexa 488 donkey α-rabbit (1:500, Invitrogen) together with Viability Dye eFluor^{®} 780 (1:10.000) each for 20 min at 4°C. Between each step cells were washed two times in DPBS with 1% BSA. FHRB binding was analyzed by flow cytometry.

### Protein binding to LDL/ MDA-LDL and C3b

5 µg/mL LDL, MDA-LDL (both Cell Biolabs) or BSA were coated 1h at 37°C, blocked with 200 µL blocking buffer I for 1h at 37°C and incubated with 30 µg/mL FHR1 2h at RT, polyclonal FHR1 antibody4 (1:1000) in Cross Down buffer (AppliChem) 1h at RT as well as HRP-labeled polyclonal anti-rabbit IgGs (Dako) 1h at RT. All steps included 3-5 wash steps with DPBS 0.05% Tween 20 (Sigma-Aldrich). Afterwards TMB (50 µL, eBioScience) was added and reaction was stopped with H2SO4 (2M, Roth). The absorbance was measured with a microplate reader at 450 nm. For the dose dependent binding MDA-LDL (5 µg/mL) were coated and after blocking 0, 1, 2.5, 5, 10 µg/mL FHR1 were incubated for 2 h 37°C. The kinetics of FHR1 binding to MDA-LDL was measured via surface plasmon resonance technique using a Biacore 3000 instrument (Biacore). MDA-LDL was immobilized to flow cells of a sensor chip (CM5) using a standard amine-coupling procedure. Different concentrations of FHR1 (250-2000 nM) diluted in PBS or PBS without protein as control were injected into the flow cells with MDA-LDL or blanc control with a flow rate of 5 µL/min at 25°C. For fragment binding studies, 5 µg/mL MDA-LDL was immobilized o.n. at 4°C. After blocking, 5 µg/mL FHR1 or 10 µg/mL FHR1 SCR 1-2 or SCR 3-5 were incubated 2h at 37°C, followed by incubation with Penta His antibody (1:1000) and polyclonal goat anti-mouse IgG (1:1000), each 1 h at RT. Afterwards TMB was added and reaction stopped with H2SO4 (2M).

To show the FHR1 binding to C3b, 5 µg/mL C3b or FHR1 were coated 1h at 37°C, blocked with blocking buffer I (200 µL) for 1h 37°C and incubated with FHR1 (10 µg/mL) for 2h at 37°C. The ELISA was continued as described for MDA-LDL.

### Replacement of FHR1 by FHR2 or FHR2DM

FHR1 SCR 1-2 (5 µg/mL) was immobilized on a microtiter plate over night at 4°C, incubated with NHS (5%) in DPBS for 1 h at 37°C, and FHR2 or FHR2DM (0, 1, 5, or 15 µg/mL) was added to each probe. FHR1 was detected by incubation with monoclonal antibody C1810 (1:1000) for 1 h at RT (between 20°C and 25°C) and FHR2DM with monoclonal FHR2 antibody9 and secondary α-mouse (1:1000). All steps included 3-5 wash steps with DPBS 0.05% Tween 20.

### Immunohistochemistrv

FFPE tissue sections were stained for presence of FHR1 (Leibniz Institute, Jena, Germany) by incubating the sections with a protease solution for 30 min at 40°C. Subsequently the sections were incubated with FHR1 antibodies (1:40 000) overnight at 4°C, and were developed using POLAP-AP (Zytomed, Berlin, Germany).

### Analysis of patient serum samples

Serum samples were collected upon informed consent. Serum levels of high sensitive IL-1β (Thermo Fisher Scientific), anti-MPO-antibodies (ORG 519 Orgentec), and anti-PR3-antibodies (ORG 618, Orgentec) were determined by ELISA according to the protocol provided by the manufacturers. CRP levels were measured by module C701 (Cobas8000).

Presence of FHR1 in NHS samples was determined by Western Blot analysis. Roti^{®}-Load 2 (2.5 µL, Roth) was added to NHS (1 µL) diluted in DPBS (10 µL) and separating the samples via SDS-PAGE. Immunoblotting 263 was performed with polyclonal FH antiserum (1:7500, CompTech) and HRP-conjugated goat antibody (1:2500, Dako).

### Statistical analysis

Significant differences between two groups were analyzed using the unpaired two tailed Student's t-test. AAV patients were analyzed using the unpaired two-tailed t test with Welch's correction. Immunohistochemistry was analyzed using Wilcoxon Signed Rank test. Correlation between IL-1β/CRP and ANCA are analyzed using Spearman correlation test. Values of * p≤0.05, ** p≤0.01, *** p≤0.001 were considered as statistically significant.

### FHR1 induces pro-inflammatory cytokine secretion by monocytes

In a previous study, it was demonstrated that FH binds to oxidized lipid deposits and inhibits complement activation and inflammatory responses (17). To investigate whether FHR1 also modulates inflammation, the inventors coated microtiter plates with FHR1 and incubated it with freshly isolated human peripheral blood monocytes in normal human serum (NHS) with or without lipopolysaccharide (LPS). Cytokine concentrations in the supernatant were measured after 20 h. The results showed that FHR1 alone strongly induced release of the pro-inflammatory cytokine IL-1β from monocytes, and increased LPS-triggered secretion of IL-1β (Fig. 1a). By contrast, immobilized FHR2, FHR3, FHL-1, FH, and BSA failed to induce IL-1β production (Fig. 1b). FHR1 induced IL-1β in a dose-dependent manner (concentrations ranging from 0.6 to 5 µg/ml) as early as 3 h after the start of co-incubation (Fig. 1c). Inflammatory responses were triggered by the C-termini of FHR1/SCR3-5 and also FH/SCR19-20, as demonstrated in a similar assay. The N-terminus (SCR1-2) of FHR1 failed to induce IL-1β (Fig. 1d). Because high levels of IL-1β secretion by monocytes can trigger a pro-inflammatory type of cell death called pyroptosis (18), the inventors measured release of the enzyme lactate dehydrogenase (LDH; a marker of cell death) into the cell supernatant. Very low concentrations of LDH were released in response to immobilized FHR1. Thus, monocytes did not undergo pyroptosis. To exclude the possibility that recombinant human FHR1 acts in a non-specific manner, the inventors also expressed the murine homolog of FHR1, called FHRB, and tested this protein in the same activation assays using isolated mouse monocytes and mouse serum. Similar to FHR1, immobilized FHRB induced IL-1β secretion by mouse monocytes. In parallel with IL-1β induction, FHR1 triggered secretion of pro-inflammatory cytokines IL-18, TNFα, and IL-6 (Fig. 1e-g), but not IL-18. It also inhibited secretion of the anti-inflammatory cytokine IL-10 by LPS-stimulated monocytes (Fig. 1h). Similar to IL-1β, TNFα was released by monocytes after 3 h of co-incubation with immobilized FHR1.

Because FHR1 circulates in human serum, the inventors next measured its pro-inflammatory function in a fluid phase. First, the microtiter plate surface was blocked with BSA. Monocytes were added followed by FHR1 and NHS. In this setting, FHR1 had no effect on IL-1β (Fig. 1i) or IL-10 production. Thus, immobilization of FHR1 was necessary to trigger the pro-inflammatory response. Furthermore, FHR1 function in terms of triggering secretion of IL-1β and IL-10 was lost without NHS, when the NHS was heat-inactivated or contained EDTA (Fig. 1j). FHR1 induced IL-1β release at low NHS concentrations (0.25%); IL-1β release increased at higher NHS concentrations (Fig. 1k). In summary, surface-bound (not free) FHR1 induces secretion of IL-1β and inhibits IL-10 release by monocytes in the presence of active NHS.

### The pro-inflammatory function of FHR1 is independent of the complement cascades

Having shown that FHR1 requires active NHS to trigger secretion of IL-1β and to inhibit IL-10 production by monocytes, the inventors asked whether the complement system is essential for its pro-inflammatory function. The inventors found that FHR1 increased secretion of IL-1β and blocked IL-10 release in the presence of C3-depleted or -inhibited NHS, (Fig. 2a). Similarly, blocking CR3, C3aR, and C5 failed to reduce FHR1-mediated IL-1β release (Fig. 2a). Neither C3 nor C5 was involved in FHR1 function. Blocking the classical- and the lectin-induced complement pathways using EGTA-treated or C1q-depleted NHS did not inhibit FHR1 activity (Fig. 2b). Similarly, blocking the alternative pathway (factor B- or P-depleted NHS) had no effect on FHR1 function (Fig. 2c). Thus, FHR1-mediated induction of IL-1β and inhibition of IL-10 secretion by monocytes is independent of the complement pathways.

### FHR1 activates NLRP3 via EMR2

Two signals induce secretion of IL-1β by monocytes. The first is triggered by DAMPs and PAMPs and is mediated, for example by Toll-like receptors. Signal 1 activates NF-κB, which then induces transcription of pro-IL-1β. Pro-IL-1β is subsequently cleaved by caspase 1 which is activated via signal 2-induced pattern recognition receptors (NLRs); the result is release of IL-1β19. To find out if FHR1-induced production of IL-1β requires both of these signals, the inventors first inhibited NF-κB and found that FHR1-mediated release of IL-1β was completely blocked (Fig. 2d). FHR1 induced transcription of pro-IL-1β and TNFα exclusively in the presence of NHS, as shown by increased RNA transcription in monocytes. Similar to NF-κB, inhibiting caspase-1 also blocked IL-1β release (Fig. 2e). However, inhibiting TLR2, TLR4, TLR6, CD14, CD36, RAGE, Dectin 1, or Fc receptors did not interfere with FHR1-induced IL-1β secretion. Thus, the inventors concluded that FHR1, together with a second signal derived from activated NHS, induces the inflammasome via a specific receptor-mediated signaling pathway.

Caspase-1 is activated by NLRP1, NLRP3, NLRC4, or AIM220. Incubating monocytes with a specific NLRP3 inhibitor completely inhibited FHR1- and FHRB- mediated IL-1β release, thereby confirming involvement of NLRP3 in the activation process (Fig. 2f). Molecules found in lysosomes, such as particulate substances or crystals of uric acid, induce formation of reactive oxygen species (ROS) or cathepsin B, which then trigger formation of the NLRP3 inflammasome. Similarly, opening of the P2X7 receptor by external ATP, which leads to potassium (K+) efflux and increased intracellular Ca2+ concentrations, also activates the NLRP3 inflammasome20. To identify the FHR1-triggered pathway, the inventors measured ROS release. FHR1 failed to induce release of ROS by monocytes (although *C. albicans* did trigger ROS release). Inhibiting K+ efflux and cathepsin B did not affect FHR1-induced IL-1β production. However, IL-1β release was blocked by phospholipase C (PLC) inhibitors (Fig. 2g) and inhibition of Gβγ, the subunit of G protein-coupled receptor (GPCR), reduced FHR1 induced IL-1β release by 65% (Fig. 2h).

As many pro-inflammatory receptors induce PLC and Ca2+, the inventors examined the signaling pathways by RNA sequencing to better identify involved receptors. FHR1-induced genes were identified in monocytes derived from four different donors upon incubation with NHS for 4 h. The inventors identified monocytic genes upregulated in response to FHR1 (gene ontology enrichment analysis: 'immune response', 'cellular response to TNF', 'neutrophil chemotaxis', 'cellular response to IL-1', and `signal transduction and inflammatory response (Fig. 2i, j)). 35 upregulated genes (7.3%) were inflammation related including NF-kB, NLRP3 and calcium-signaling pathways (KEGG) (Fig. 2k, l). Gene associated disease (GAD) analysis confirmed that patterns of upregulated genes by FHR1 are found in chronic renal failure, hypertension, myocardial infarction, atherosclerosis, stroke and blood pressure.

Furthermore FHR1 upregulated the G protein-coupled receptor EMR2/ADGRE2, which was previously described to induce an inflammatory response in macrophages via PLC and intracellular Ca2+ release (21). Inhibition of the EMR2 receptor indeed blocked FHR1 induced IL-1β (Fig. 2m) release. Thus, FHR1 induces together with a serum factor NF-κB-dependent transcription of pro-IL-1β. Via GPCR EMR2 FHR1 activates PLC, which cleaves phosphatidylinositol (PPI) to inositoltriphosphate (IP3) and thereby stimulates Ca2+ release from the endoplasmic reticulum which activates NLRP3, followed by activation of caspase-1, which then cleaves pro-IL-1β to IL-1β22 (Fig. 2n).

### FHR1 binds to necrotic cells and induces secretion of IL-1β by monocytes

Having shown that surface-coated FHR1 induces the NLRP3 inflammasome, the inventors asked whether FHR1 also mediates this function by binding naturally to cell surfaces. Human healthy or necrotic endothelial cells (HUVECs) were incubated with FHR1 or purified FH, and binding was analyzed by flow cytometry. FHR1, but not FH, bound to necrotic HUVECs and neither molecule bound to healthy cells (Fig. 3a). Similar to human FHR1, FHRB bound to necrotic mouse alveolar macrophages, but not to healthy cells. FHR1 binding to necrotic cells was confirmed by incubating necrotic HUVECs with FHR1 and staining with a monoclonal antibody specific for FHR1 together with wheat germ agglutinin (WGA Texas Red) to lighten the cellular surface. FHR1 bound to distinct spots on necrotic cells, which had lost cell surface integrity (as seen by reduced WGA staining) (Fig. 3b). When necrotic HUVECs were incubated in NHS, both FH and FHR1 from NHS bound to necrotic but not to living cells (Fig. 3c). In this case, binding of FH was explained by its interaction with C3b on the surface; by contrast, FHR1 bound to cells in the presence/absence of C3b. When bound to C3b via SCR3-58, FHR1 lost the ability to induce release of IL-1β (Fig. 3d). However, FHR1 alone attached to necrotic cells via the N-terminal SCR1-2 domains, as shown by flow cytometry. Therefore, FHR1 domains SCR3-5 trigger the inflammatory response when FHR1 bound to necrotic cells.

To examine whether necrotic cell surface-bound FHR1 also modulates cytokine responses of monocytes, the inventors incubated necrotic HUVECs with FHR1 (10 µg). After extensive washing, these cells were incubated with monocytes for 20 h in the presence of NHS. FHR1 bound to necrotic cells and triggered a >4-fold increase of IL-1β release by monocytes when compared with that by untreated necrotic cells (Fig. 3e). As expected, incubating necrotic cells with monocytes in NHS containing FHR1 triggered IL-1β release. The IL-1β level dropped by around 60% when monocytes were incubated with FHR1 deficient (ΔFHR1/3) NHS (Fig. 3f). Reconstitution of ΔFHR1/3 serum with FHR1 resulted in attachment of FHR1 to the necrotic cells and strong release of IL-1β by monocytes; this was not the case for FH (Fig. 3g).

### FHR1 binds to MDA-LDL and increases release of IL-1β by monocytes

Malondialdehyd-modified low density lipoproteins (MDA-LDL) are a marker of oxidative stress and are produced by damaged and necrotic cells (23). FHR1 binds to MDA-LDL, but not to LDL or BSA (Fig. 3h), in a dose-dependent manner. Binding of FHR1 to MDA-epitopes expressed by necrotic HUVECs was confirmed using proximity ligation assay (Fig. 3i). Similar to necrotic cells, FHR1 also bound to MDA-LDL via SCR1-2 (Fig. 3j). MDA-LDL-bound FHR1 increased secretion of IL-1β by monocytes by about 40% (Fig. 3k). Thus, FHR1 is a sensor of necrotic cell surfaces and induces inflammation.

### FHR1 binds to necrotic cells in vivo

The inventors next asked whether FHR1 is targeted to specific necrotic cells *in vivo.* Necrosis and inflammation are hallmarks of AAV and AS (24, 25). Therefore, attachment of FHR1 to atherosclerotic plaques in AS, and to glomeruli in AAV patients was examined by immunohistochemistry (IHC). Plaques on surfaces are formed by lipid accumulation and oxidation; these plaques contain necrotic cells. Staining of human plaques in the human artery and heart valve with a monoclonal antibody specific for FHR1, which does not recognize FH, revealed specific binding of FHR1 to the plaques, but not to healthy cells close to the plaques (Fig. 4a, b). In contrast to FHR1 FHR2 showed low binding to the heart valve and is found predominantly in the intima of the artery (Fig. 4a, b).

In AAV, necrotizing vasculitis leads to endothelial cell damage, and necrotic cells are present particularly in small blood vessels in the glomeruli, which cause crescentic glomerulonephritis (26). Therefore, kidney tissue sections from patients diagnosed with AAV and nephritis were stained for FHR1. FHR1 was present on glomerular crescents composed of necrotic cells but once again not on healthy tissue (Fig. 4c).

### Serum concentrations of IL-1β in patients with vasculitis

FHR1 binds to necrotic cells and induces inflammation. To determine the relevance of this process, the inventors measured IL-1β concentrations in patients with AAV and separated them into two groups according to the presence or absence of FHR1. In a cohort of 314 AAV patients, 11 had ΔFHR1/3 deficiency, which is consistent with the frequency in the normal healthy Caucasian population (27). AAV patients harboring FHR1/3 or ΔFHR1/3 are of similar age and gender. When the inventors compared IL-1β serum concentrations according to the presence/absence of FHR1, the inventors found very low amounts of IL-1β in AAV patients with ΔFHR1/3 (0.6±0.1 pg/ml), similar to those in healthy controls (0.4±0.1 pg/ml). By contrast, in patients with active FHR1 significantly higher IL-1β concentrations were detected (1.4±0.3 pg/ml, p<0.05) than in ΔFHR1/3 patients (Fig. 4d). To confirm FHR1-dependent regulation of IL-1β, serum samples were collected from AS patients. 3 of 124 patients within this cohort harbored ΔFHR1/3 (2.4%). Both groups were of similar age and gender. The IL-1β level in AS samples in general was not significantly higher compared to the healthy controls, and did not allow comparison between patients with or without FHR1. IL-1β serum concentrations did not directly correlate with ANCA antibodies, in contrast to C-reactive protein (CRP). Therefore, serum CRP concentrations, which can substantially increase in response to IL-6, were analyzed in serum samples of AAV patients and revealed significant lower amounts in samples from patients with ΔFHR1/3 (2.8 mg/dL ± 0.8 mg/dL) in comparison to patients harboring FHR1/3 (36 mg/dL ± 3.5 mg/dL, p<0.001) (Fig. 4e). Similarly, AS patients with ΔFHR1/3 showed low amounts of CRP (Fig. 4f). Altogether the data demonstrate an *in vivo* influence of FHR1 on inflammation.

### FHR2 inhibits FHR1

FHR1 forms homodimers and heterodimers with FHR2; also FHR1 is very flexible in forming homo and heterodimers (10). When SCR1-2 of FHR1 was immobilized and incubated with NHS, the inventors found that serum-derived FHR1 bound to FHR1 SCR1-2. Addition of increasing amounts of FHR2 out-competed serum-bound FHR1 (Fig. 4g). By contrast, replacement of FHR1 was not observed when the dimerization motif of FHR2 was mutated (FHR2DM) (Fig. 4h). The inventors next examined whether FHR2 also inhibits the pro-inflammatory activity of necrotic cell attached FHR1 by adding FHR2 to ΔFHR1/3 serum together with monocytes. As expected, addition of FHR2, but not FHR2DM, to the serum reduced the concentration of IL-1β by about 50% (Fig. 4i, j). These results demonstrate that FHR2 inhibits FHR1 activity by forming heterodimers, and that FHR2 may restrict FHR1-mediated inflammation.

### References as cited

1. Chen, M. et al. Antineutrophil cytoplasmic autoantibody - negative Pauci-immune crescentic glomerulonephritis. Journal of the American Society of Nephrology: JASN 18, 599-605 (2007).
2. Zheng, F., Xing, S., Gong, Z. & Xing, Q. NLRP3 inflammasomes show high expression in aorta of patients with atherosclerosis. Heart, lung & circulation 22, 746-750 (2013).
3. Baldrighi, M., Mallat, Z. & Li, X. NLRP3 inflammasome pathways in atherosclerosis. Atherosclerosis 267, 127-138 (2017).
4. Lotze, M.T. et al. The grateful dead: damage-associated molecular pattern molecules and reduction/oxidation regulate immunity. Immunological reviews 220, 60-81 (2007).
5. Bosurgi, L., Manfredi, A.A. & Rovere-Querini, P. Macrophages in injured skeletal muscle: a perpetuum mobile causing and limiting fibrosis, prompting or restricting resolution and regeneration. Frontiers in immunology 2, 62 (2011).
6. Jennette, J.C. et al. 2012 revised International Chapel Hill Consensus Conference Nomenclature of Vasculitides. Arthritis and rheumatism 65, 1-11 (2013).
7. Zipfel, P.F. & Skerka, C. Complement regulators and inhibitory proteins. Nature reviews. Immunology 9, 729-740 (2009).
8. Heinen, S. et al. Factor H-related protein 1 (CFHR-1) inhibits complement C5 convertase activity and terminal complex formation. Blood 114, 2439-2447 (2009).
9. Skerka, C., Horstmann, R.D. & Zipfel, P.F. Molecular cloning of a human serum protein structurally related to complement factor H. The Journal of biological chemistry 266, 12015-12020 (1991).
10. van Beek, A.E. et al. Factor H-Related (FHR)-1 and FHR-2 Form Homo- and Heterodimers, while FHR-5 Circulates Only As Homodimer in Human Plasma. Frontiers in immunology 8, 1328 (2017).
11. Goicoechea de Jorge, E. et al. Dimerization of complement factor H-related proteins modulates complement activation in vivo. Proceedings of the National Academy of Sciences of the United States of America 110, 4685-4690 (2013).
12. Park, C.T. & Wright, S.D. Plasma lipopolysaccharide-binding protein is found associated with a particle containing apolipoprotein A-I, phospholipid, and factor H-related proteins. The Journal of biological chemistry 271, 18054-18060 416 (1996).
13. Gharavi, A.G. et al. Genome-wide association study identifies susceptibility loci for IgA nephropathy. Nature genetics 43, 321-327 (2011).
14. Hughes, A.E. et al. A common CFH haplotype, with deletion of CFHR1 and CFHR3, is associated with lower risk of age-related macular degeneration. Nature genetics 38, 1173-1177 (2006).
15. Zhao, J. et al. Association of genetic variants in complement factor H and factor H-related genes with systemic lupus erythematosus susceptibility. PLoS genetics 7, e1002079 (2011).
16. Zipfel, P.F. et al. Deletion of complement factor H-related genes CFHR1 and CFHR3 is associated with atypical hemolytic uremic syndrome. PLoS genetics 3, e41 (2007).
17. Weismann, D. et al. Complement factor H binds malondialdehyde epitopes and protects from oxidative stress. Nature 478, 76-81 (2011).
18. Fink, S.L. & Cookson, B.T. Caspase-1-dependent pore formation during pyroptosis leads to osmotic lysis of infected host macrophages. Cellular microbiology 8, 1812-1825 (2006).
19. Thornberry, N.A. et al. A novel heterodimeric cysteine protease is required for interleukin-1 beta processing in monocytes. Nature 356, 768-774 (1992).
20. Aziz, M., Jacob, A. & Wang, P. Revisiting caspases in sepsis. Cell death & disease 5, e1526 (2014).
21. I, K.-Y. et al. Activation of Adhesion GPCR EMR2/ADGRE2 Induces Macrophage Differentiation and Inflammatory Responses via Gα16/Akt/MAPK/NF-κB Signaling Pathways. Frontiers in immunology 8, 373 (2017).
22. Lee, G.-S. et al. The calcium-sensing receptor regulates the NLRP3 inflammasome through Ca2+ and cAMP. Nature 492, 123-127 (2012).
23. Chang, M.K. et al. Monoclonal antibodies against oxidized low-density lipoprotein bind to apoptotic cells and inhibit their phagocytosis by elicited macrophages: evidence that oxidation-specific epitopes mediate macrophage recognition. Proceedings of the National Academy of Sciences of the United States of America 96, 6353-6358 (1999).
24. Yates, M. & Watts, R. ANCA-associated vasculitis. Clinical medicine (London, England) 17, 60-64 (2017).
25. Martinet, W., Schrijvers, D.M. & De Meyer, Guido R Y. Necrotic cell death in atherosclerosis. Basic research in cardiology 106, 749-760 (2011).
26. Jennette, J.C., Falk, R.J. & Gasim, A.H. Pathogenesis of antineutrophil cytoplasmic autoantibody vasculitis. Current opinion in nephrology and hypertension 20, 263-270 (2011).
27. Holmes, L.V. et al. Determining the population frequency of the CFHR3/CFHR1 456 deletion at 1q32. PloS one 8, e60352 (2013).
28. Manthiram, K., Zhou, Q., Aksentijevich, I. & Kastner, D.L. The monogenic autoinflammatory diseases define new pathways in human innate immunity and inflammation. Nature immunology 18, 832-842 (2017).
29. Celkova, L., Doyle, S.L. & Campbell, M. NLRP3 Inflammasome and Pathobiology in AMD. Journal of clinical medicine 4, 172-192 (2015).
30. Binder, C.J., Papac-Milicevic, N. & Witztum, J.L. Innate sensing of oxidation-specific epitopes in health and disease. Nature reviews. Immunology 16, 485-464 497 (2016).
31. Obermayer, G., Afonyushkin, T. & Binder, C.J. Oxidized low-density lipoprotein in inflammation-driven thrombosis. Journal of thrombosis and haemostasis : JTH 16, 418-428 (2018).
32. Okemefuna, A.I., Nan, R., Gor, J. & Perkins, S.J. Electrostatic interactions contribute to the folded-back conformation of wild type human factor H. Journal of molecular biology 391, 98-118 (2009).
33. Hannan, J.P., Laskowski, J., Thurman, J.M., Hageman, G.S. & Holers, V.M. Mapping the Complement Factor H-Related Protein 1 (CFHR1):C3b/C3d Interactions. PloS one 11, e0166200 (2016).
34. Huang, C.-H. et al. Increased EMR2 expression on neutrophils correlates with disease severity and predicts overall mortality in cirrhotic patients. Scientific reports 6, 38250 (2016).
35. van Eijk, M. et al. Differential expression of the EGF-TM7 family members CD97 478 and EMR2 in lipid-laden macrophages in atherosclerosis, multiple sclerosis and 479 Gaucher disease. Immunology letters 129, 64-71 (2010).
36. Stone, J.H. et al. Rituximab versus cyclophosphamide for ANCA-associated vasculitis. The New England journal of medicine 363, 221-232 (2010).
37. Yates, M. et al. EULAR/ERA-EDTA recommendations for the management of ANCA-associated vasculitis. Annals of the rheumatic diseases 75, 1583-1594 (2016).
38. Ridker, P.M. et al. Antiinflammatory Therapy with Canakinumab for Atherosclerotic Disease. The New England journal of medicine 377, 1119-1131 (2017).
39. Fritsche, L.G. et al. An imbalance of human complement regulatory proteins 489 CFHR1, CFHR3 and factor H influences risk for age-related macular degeneration (AMD). Human molecular genetics 19, 4694-4704 (2010).
40. Hageman, G.S. et al. Extended haplotypes in the complement factor H (CFH) and CFH-related (CFHR) family of genes protect against age-related macular degeneration: characterization, ethnic distribution and evolutionary implications. Annals of medicine 38, 592-604 (2006).
41. Józsi, M. et al. Factor H autoantibodies in atypical hemolytic uremic syndrome correlate with CFHR1/CFHR3 deficiency. Blood 111, 1512-1514 (2008).
42. Foltyn Zadura, A. et al. Factor H autoantibodies and deletion of Complement Factor H-Related protein-1 in rheumatic diseases in comparison to atypical hemolytic uremic syndrome. Arthritis research & therapy 14, R185 (2012).

## Claims

1. An in vitro Or method for identifying a compound that modulates sterile inflammation through the modulation of the interaction of FHR1 with EMR2/ADGRE2 in a cell, comprising the steps of
a) contacting at least one of FHR1, an EMR2/ADGRE2 binding fragment of FHR1, a cell expressing FHR1, and/or a cell expressing an EMR2/ADGRE2 binding fragment of FHR1 with at least one compound that potentially modulates the interaction of FHR1 with EMR2/ADGRE2 in a cell
b) identifying a modulation of the binding of FHR1 or said fragment to EMR2/ADGRE2 in the presence of said at least one compound, and
c) identifying a compound as identified in step b) as specifically modulating sterile inflammation.

2. The method according to claim 1, wherein said modulation is selected from a decrease or an increase of said binding of FHR1 to EMR2/ADGRE2.

3. The method according to claim 1 or 2, wherein said identifying comprises a method selected from rtPCR, immunoprecipitation and/or a measuring the induction or reduction of inflammation in said cell, for example by detecting cytokine IL-1β, IL-6, IL-18, C-reactive protein (CRP) and/or TNFα.

4. The method according to any one of claims 1 to 3, wherein said compound is selected from the group consisting of a peptide library, a combinatory library, a cell extract, a "small molecular drug", an antisense oligonucleotide, an siRNA, and an antibody, in particular an antibody against FHR1 or fragment thereof, e.g. a F(ab)₂-fragment, specifically interfering with the binding of FHR1 to EMR2/ADGRE2, FHR2 or binding fragment thereof, and a receptor blocker, such as an NLRP3 inhibitor.

5. The method according to any one of claims 1 to 4, wherein said method comprises the use of human peripheral blood monocytes, normal human serum (NHS), and/or lipopolysaccharide (LPS).

6. The method according to any one of claims 1 to 5, wherein said EMR2/ADGRE2 binding fragment of FHR1 comprises the C-terminus FHR1/SCR3-5 or the N-terminus FHR1/SCR1-2 of the FHR1.

7. The method according to any one of claims 1 to 6, further comprising testing said compound as identified for its activity to induce or reduce sterile inflammatory responses.

8. The method according to any one of claims 1 to 7, further comprising a computational analysis of and optimization of said compound based on the structure, in particular the three-dimensional and/or crystal structure of FHR1 and/or EMR2/ADGRE2.

9. Use of an isolated cell expressing FHR1, and/or expressing an EMR2/ADGRE2 binding fragment thereof, wherein said cell optionally expresses EMR2/ADGRE2 and/or an FHR1 binding fragment thereof, as a screening tool for screening for a compound that modulates the interaction of FHR1 with EMR2/ADGRE2 on/in a cell.

10. The use according to claim 9, wherein said FHR1 and/or EMR2/ADGRE2 and/or the fragments thereof are immobilized and/or labeled.

11. The use according to claim 9 or 10, wherein said EMR2/ADGRE2 binding fragment of FHR1 comprises the C-terminus of FHR1/SCR3-5 or the N-terminus FHR1/SCR1-2 of the FHR1 polypeptide.

## Patentansprüche

1. In vitro Verfahren zur Identifizierung einer Verbindung, die sterile Entzündung durch die Modulation der Interaktion von FHR1 mit EMR2/ADGRE2 in einer Zelle moduliert, umfassend die Schritte von
a) in Kontakt bringen von mindestens einem von FHR1, einem EMR2/ADGRE2 bindenden Fragment von FHR1, einer Zelle die FHR1 exprimiert, und/oder einer Zelle die ein EMR2/ADGRE2 bindendes Fragment von FHR1 exprimiert, mit mindestens einer Verbindung die potentiell die Interaktion von FHR1 mit EMR2/ADGRE2 in einer Zelle moduliert,
b) Identifizieren einer Modulation der Bindung von FHR1 oder des Fragments an EMR2/ADGRE2 in der Anwesenheit der mindestens einen Verbindung, und
c) Identifizieren einer Verbindung wie in Schritt b) identifiziert als spezifisch die sterile Entzündung modulierend.

2. Verfahren nach Anspruch 1, wobei die Modulation ausgewählt ist aus einer Abnahme oder einer Zunahme der Bindung von FHR1 an EMR2/ADGRE2.

3. Verfahren nach Anspruch 1 oder 2, wobei das Identifizieren ein Verfahren ausgewählt aus rtPCR, Immunpräzipitation und/oder ein Messen der Induktion oder Reduktion von Entzündung in der Zelle umfasst, zum Beispiel durch Nachweisen von Zytokin IL-1β, IL-6, IL-18, C-reaktivem Protein (CRP) und/oder TNFα.

4. Verfahren nach einem der Ansprüche 1 bis 3, wobei die Verbindung ausgewählt ist aus der Gruppe bestehend aus einer Peptidbibliothek, einer kombinatorischen Bibliothek, einem Zellextrakt, einem "Wirkstoff mit kleinem Molekulargewicht", einem antisense Oligonukleotid, einer siRNA und einem Antikörper, insbesondere einem Antikörper gegen FHR1 oder einem Fragment davon, z.B. einem F(ab)₂-Fragment, das spezifisch mit der Bindung von FHR1 an EMR2/ADGRE2 interferiert, FHR2 oder einem bindenden Fragment davon, und einem Rezeptorblocker, wie zum Beispiel einem NLRP3 Inhibitor.

5. Verfahren nach einem der Ansprüche 1 bis 4, wobei das Verfahren die Verwendung von menschlichen peripheren Blutmonozyten, normalem menschlichem Serum (NHS), und/oder Lipopolysaccharid (LPS) umfasst.

6. Verfahren nach einem der Ansprüche 1 bis 5, wobei das EMR2/ADGRE2 bindende Fragment von FHR 1 den C-Terminus FHR1/SCR3-5 oder den N-Terminus FHR1/SCR1-2 des FHR1 Polypeptids umfasst.

7. Verfahren nach einem der Ansprüche 1 bis 6, weiter umfassend ein Testen der Verbindung wie identifiziert auf ihre Aktivität, sterile Entzündungsantworten zu induzieren oder zu reduzieren.

8. Verfahren nach einem der Ansprüche 1 bis 7, weiter umfassend eine computergestützte Analyse von und optimieren der Verbindung basierend auf der Struktur, insbesondere der dreidimensionalen und/oder Kristallstruktur von FHR1 und/oder EMR2/ADGRE2.

9. Verwendung einer isolierten Zelle die FHR1 exprimiert, und/oder ein EMR2/ADGRE2 bindendes Fragment davon exprimiert, wobei die Zelle gegebenenfalls EMR2/ADGRE2 und/oder ein FHR1 bindendes Fragment davon exprimiert, als ein Screeningtool zum Screenen auf eine Verbindung die die Interaktion von FHR1 mit EMR2/ADGRE2 auf/in einer Zelle moduliert.

10. Verwendung nach Anspruch 9, wobei das FHR1 und/oder EMR2/ADGRE2 und/oder die Fragmente davon immobilisiert und/oder markiert sind.

11. Verwendung nach Anspruch 9 oder 10, wobei das EMR2/ADGRE2 bindende Fragment von FHR 1 den C-Terminus FHR1/SCR3-5 oder denN-Terminus FHR1/SCR1-2 des FHR1 Polypeptids umfasst.

## Revendications

1. Une méthode in vitro pour identifier un composé qui module l'inflammation stérile par la modulation de l'interaction de FHR1 avec EMR2/ADGRE2 dans une cellule, comprenant les étapes de
a) mise en contact d'au moins un de FHR1, un fragment de liaison EMR2/ADGRE2 de FHR1, une cellule exprimant FHR1 et/ou une cellule exprimant un fragment de liaison EMR2/ADGRE2 de FHR1 avec au moins un composé qui module potentiellement l'interaction de FHR1 avec EMR2/ADGRE2 dans une cellule
b) identification d'une modulation de la liaison de FHR1 ou dudit fragment à EMR2/ADGRE2 en présence dudit au moins un composé, et
c) identification d'un composé identifié à l'étape b) comme modulant spécifiquement l'inflammation stérile.

2. Méthode selon la revendication 1, dans laquelle ladite modulation est choisie parmi une diminution ou une augmentation de la liaison de FHR1 à EMR2/ADGRE2.

3. Méthode selon la revendication 1 ou 2, dans laquelle ladite identification comprend une méthode choisie parmi la rtPCR, l'immunoprécipitation et/ou une mesure de l'induction ou de la réduction de l'inflammation dans ladite cellule, par exemple en détectant la cytokine IL-113, IL-6, IL-18, la protéine C-réactive (CRP) et/ou le TNFα.

4. Méthode selon l'une quelconque des revendications 1 à 3, dans laquelle ledit composé est choisi dans le groupe constitué d'une bibliothèque de peptides, d'une bibliothèque combinatoire, d'un extrait cellulaire, d'un "petit médicament moléculaire", d'un oligonucléotide antisens, d'un siRNA et d'un anticorps, en particulier un anticorps contre FHR1 ou un de ses fragments, par exemple un fragment F(ab)₂ , interférant spécifiquement avec la liaison de FHR1 à EMR2/ADGRE2, FHR2 ou un fragment de liaison de celui-ci, et un bloqueur de récepteur, tel qu'un inhibiteur de NLRP3.

5. Méthode selon l'une quelconque des revendications 1 à 4, dans laquelle ladite méthode comprend l'utilisation de monocytes de sang périphérique humain, de sérum humain normal (SHN) et/ou de lipopolysaccharide (LPS).

6. Méthode selon l'une quelconque des revendications 1 à 5, dans laquelle ledit fragment de liaison EMR2/ADGRE2 de FHR1 comprend l'extrémité C-terminale FHR1/SCR3-5 ou l'extrémité N-terminale FHR1/SCRI-2 du polypeptide FHR1.

7. Méthode selon l'une quelconque des revendications 1 à 6, comprenant en outre de tester le composé identifié pour son activité d'induction ou de réduction des réponses inflammatoires stériles.

8. Méthode selon l'une quelconque des revendications 1 à 7, comprenant en outre une analyse computationnelle et une optimisation dudit composé sur la base de la structure, en particulier la structure tridimensionnelle et/ou cristalline de FHR1 et/ou EMR2/ADGRE2.

9. Utilisation d'une cellule isolée exprimant FHR1, et/ou exprimant un fragment de liaison EMR2/ADGRE2, dans laquelle ladite cellule exprime optionnellement EMR2/ADGRE2 et/ou un fragment de liaison FHR1, comme outil de criblage pour la recherche d'un composé qui module l'interaction de FHR1 avec EMR2/ADGRE2 sur/dans une cellule.

10. Utilisation selon la revendication 9, dans laquelle ledit FHR1 et/ou EMR2/ADGRE2 et/ou leurs fragments sont immobilisés et/ou marqués.

11. Utilisation selon la revendication 9 ou 10, dans laquelle ledit fragment de liaison EMR2/ADGRE2 de FHR1 comprend l'extrémité C-terminale de FHR1/SCR3-5 ou l'extrémité N-terminale de FHR1/SCRI-2 du polypeptide FHR1.
